# EUROPÄISCHE PATENTSCHRIFT

(11) **EP 3 513 675 B1**
(45) Veröffentlichungstag und Bekanntmachung des Hinweises auf die Patenterteilung: **13.05.2020**
(21) Anmeldenummer: 18152947.0
(22) Anmeldetag: 23.01.2018
(51) Int. Cl.: A43B 7/14, A43B 17/00, B29D 35/14

(54) **ORTHOPÄDISCHE SCHAUMKUNSTSTOFF-SCHUHEINLAGE**
ORTHOPEDIC FOAM PLASTIC SHOE INSERT
INSERT DE CHAUSSURE DE MOUSSE EN MATIÈRE PLASTIQUE ORTHOPÉDIQUE

(43) Veröffentlichungstag der Anmeldung: 24.07.2019
(73) Patentinhaber: Spannrit GmbH, 63801 Kleinostheim (DE)
(72) Erfinder: KATZER, Roland, 63801 Kleinostheim (DE)
(74) Vertreter: Erhardt, Martin

(56) Entgegenhaltungen:
- EP-A1- 1 516 715
- EP-A1- 2 910 677
- EP-A1- 2 918 185

## Beschreibung

Die Erfindung betrifft orthopädische Schaumkunststoff-Schuheinlagen, insbesondere orthopädische Schaumkunststoff-Schuheinlagen, welche im RIM-Schäumverfahren hergestellt werden. Weiter betrifft die vorliegende Erfindung insbesondere Schaumkunststoff-Schuheinlagen mit einem im RIM-Schäumverfahren erzeugten orthopädischen Fußbett auf der Oberseite der Schuheinlage. Die erfindungsgemäße Schuheinlage bzw. das erfindungsgemäße Verfahren zum Herstellen einer solchen Schuheinlage, weist weiter eine Unterdecke sowie gegebenenfalls eine Oberdecke zur Verbesserung des Tragekomforts auf. Weiterhin weist die erfindungsgemäße Schuheinlage gemäß Anspruch 13 ein im Spritzgussverfahren hergestelltes, die Schuheinlage stabilisierendes Kernteil auf. Im Stand der Technik werden gattungsgemäße Schaumkunststoff-Schuheinlagen mit einem sohlenstabilisierenden Kernteil gemäß Oberbegriff des Anspruchs 13 häufig im RIM-Schäumverfahren hergestellt, wobei ein vorgefertigtes Kernteil in die RIM-Schäumform eingelegt wird und dort, beispielsweise mittels Stiften, die in das Kernteil ragen, fixiert. Diese Vorgehensweise bei der Herstellung einer Schaumkunststoff-Schuheinlage wird beispielsweise in EP 1 602 294 A1 erläutert, bei der das Kernteil allseitig mit PU-Schaumkunststoff im RIM-Verfahren umschäumt bzw. ummantelt wird. Nach dem Aushärten des PU-Schaumkunststoffes wird der PU-Rohling aus der Ausschäumform entnommen und kann anschließend sowohl oberseitig als auch unterseitig mit einer Decke versehen werden. Diese Decken werden üblicherweise mit einem (Heiß)-Kleber mit dem PU-Schaumkunststoff-Rohling / -Polsterkörper verbunden. Jedoch sind diese Schritte für das Aufklebens einer Unterdecke und/oder einer Oberdecke auf den PU-Schaumkunststoff-Rohling nicht nur zusätzliche Arbeitsschritte, die zusätzliche spezifische Arbeitsvorrichtungen benötigen, sondern im Allgemeinen auch Arbeitsschritte, die viel Sorgfalt im Umgang mit dem Kleber erfordern, damit eine qualitativ hochwertige Schuheinlage entsteht. Das vollständig ummantelte Kernteil gemäß EP 1 602 294 A1 ist aufgrund seiner vollständigen Ummantelung mit PU-Schaumkunststoff nachträglich beispielsweise durch einen Schuhorthopädietechniker nicht mehr individuell an die Bedürfnisse des Trägers der Schuheinlage anpassbar. Zur Vermeidung der vollständigen Ummantelung schlägt EP 1 967 086 A1 vor, die Außenseite des Kernteils so auszugestalten, dass der dort angeschäumte PU-Schaumstoff nur so leicht daran anhaftet, dass dieser händisch einfach entfernbar ist. Somit kann auf einfache Art und Weise eine Außenseite des Kernteils freigelegt und somit bearbeitbar gemacht werden.

Um auch diesen Schritt des Freilegens des Kernteils zu vermeiden, schlägt EP 2 241 207 A1 als Weiterentwicklung des vorgenannten Standes der Technik vor, das sohlenstabilisierende Kernteil mit seiner Außenseite direkt auf die formgebende Innenseite der PU-Schäumform zu legen. Damit das Kernteil gemäß diesem Stand der Technik während des RIM-Schäumvorganges in der RIM-Schäumform nicht aufschwimmt, wird das Kernteil in seinen transversalen Abmessungen quer zur Kavität der Schäumform, d.h. quer zur Schließrichtung, größer vorgeformt, damit es mit einem Presssitz in der Formhälfte der RIM-Schäumform gehalten wird. Hierzu wird das Kernteil bevorzugt in einem ersten PU-Schäumschritt vorgeformt. Um das geschäumte Kernteil zum Erreichen ausreichend hoher Querkräfte für einen prozessstabilen Presssitz des Kernteils mit ausreichend Elastizität in einer zweiten RIM-Schäumform festsetzen zu können, muss dieses in Form-Schließrichtung, also in Sohlen-Dickenrichtung entsprechend dick ausgelegt sein.

In einer zweiten Schäumform wird das Kernteil mit PU-Schaumkunststoff angeschäumt und so die fertige Schuheinlage im RIM-Verfahren geschäumt. Dies führt im Ergebnis zu einer relativ dicken Schaumkunststoff-Schuheinlage die, bevor diese in einen Schuh eingelegt werden kann, oft die Entnahme der Schuhinnensohle erfordert, damit insbesondere die Fersenaufnahme des Schuhs nicht verloren geht oder andere biometrische Eigenschaften des Schuhs nicht verändert bzw. negativ beeinträchtigt werden.

Nach Entnahme eines so hergestellten PU-Schaumkunststoff-Schuheinlagenrohlings mit geschäumten, sohlenstabilisierenden Kernteil kann dieser Rohling, ebenso wie der Rohling mit dem vollständig umschäumten Kernteil gemäß EP 1 602 294 A1, auf herkömmliche Art und Weise auf der jeweiligen Ober- und/oder Unterseite mittels eines Klebeschrittes mit einer entsprechenden Oberdecke bzw. Unterdecke bezogen werden. Des Weiteren offenbaren die Dokumente EP 2 918 185 A1, EP 1 516 715 A1 und EP 2 910 677 A1 ebenfalls orthopädische Einlagen mit einem Kernteil und Verfahren zum Herstellen von orthopädischen Schuheinlagen.

Aufgabe der Erfindung ist es daher, ein vereinfachtes Herstellverfahren für eine solche im RIM-Verfahren herstellbare Schaumkunststoff-Schuheinlage mit einem sohlenstabilisierenden Kernteil bereitzustellen, wobei einerseits das Herstellverfahren insgesamt vereinfacht wird und andererseits der zusätzliche und viel Sorgfalt erfordernde Aufwand für das Beziehen/Bekleben eines PU-Schaumkunststoff-Rohlings mit einer Unterdecke und gegebenenfalls mit einer Oberdecke auf ein Minimum reduziert wird. Mittels der Erfindung soll möglichst die Anzahl der erforderlichen Verfahrensschritte, der spezifischen Aufnahmen, der Spezialwerkzeuge und Spezialformen zum Ausbilden der Schaumkunststoff-Schuheinlage reduziert werden. In einer Ausführungsform soll ferner einem Schuhorthopädietechniker ermöglicht werden, das Kernteil der erfindungsgemäßen PU-Schuheinlage den Bedürfnissen des Trägers der Schuheinlage anpassen zu können. Mit dem erfindungsgemäßen Herstellverfahren sollen weiter Wandstärken-reduzierte Schaumkunststoff-Schuheinlagen fertigbar sein, die möglichst ohne nennenswerte Veränderung der biometrischen Verhältnisse in einen Schuhe einlegbar sind, ohne dass Veränderungen am Schuh, wie beispielsweise die Entnahme der Schuhinnensohle, erforderlich sind. Darüber hinaus soll das erfindungsgemäße Verfahren zum Herstellen einer PU-Schaumkunststoff-Schuheinlage möglichst einfach in seiner Ausführung, kostengünstig sowie robust sein, um damit qualitativ hochwertige, orthopädisch wirksame Schaumkunststoff-Schuheinlagen kostengünstig herstellen zu können.

Die Erfindung wird in den Hauptansprüchen definiert und charakterisiert, während die abhängigen Ansprüche weitere Merkmale der Erfindung beschreiben.

Aus den vorgenannten Schritten zur Herstellung einer erfindungsgemäßen, orthopädischen PU-Schaumkunststoff-Schuheinlage erkennt der Fachmann ohne Weiteres, dass zunächst im Spritzgussverfahren ein orthopädisch wirksames Kernteil hergestellt werden kann, wodurch insbesondere die Wandstärke in Dickenrichtung der späteren Schuheinlage je nach verwendetem Spritzgussmaterial dünn oder sogar sehr dünn ausgestaltet sein kann. Speziell die im Stand der Technik aus PU-Schaumkunststoff gefertigten Kernteile/Einlegeteile mussten zum Bereitstellen neben der orthopädischen Funktion, insbesondere auch für deren Presssitz (siehe oben) in der Schäumform, für die fertige PU-Schuheinlage mit relativ großen Wandstärken ausgebildet werden. Dies zum einen deshalb, damit das Kernteil einen die Schuheinlage stabilisierenden und somit auch orthopädischen Effekt aufweist und zum anderen, damit das geschäumte Kernteil genügend hohe elastische Kräfte zum Halten des Kernteils mit Presssitz in der Schäumform erzeugen kann.

Erfindungsgemäß können durch Spritzgießen des Kernteils die orthopädisch wichtigen Funktionen des sohlenstabilisierenden und fußstützenden Kernteils schon mit sehr geringen Wandstärken erreicht werden. Durch die erfindungsgemäße Positionierung und Fixierung des Kernteils an einer ersten Lage kann das Kernteil zusammen mit der ersten Lage in die RIM-Schäumform in der orthopädisch vorbestimmten Position eingelegt und dort fixiert werden. Ein Festsetzen des Kernteils durch Stifte oder mittels Presssitz ist nicht länger erforderlich. Auch bei gleichbleibender Polsterschichtdicke im Vergleich zu herkömmlichen Schuheinlagen, kann bereits so eine insgesamt dünnere Schaumkunststoff-Schuheinlage hergestellt werden, die dennoch alle orthopädischen Anforderungen, insbesondere hinsichtlich Stütz- und Polsterwirkung, erfüllt.

Bevorzugt bildet in einer Ausführungsform die erste Lage gleichzeitig die Unterdecke aus. Hierzu ist die erste Lage beispielsweise mit einer für PU-Schaumkunststoff undurchlässigen Schicht versehen oder besteht aus einem für Schaumkunststoff undurchlässigen Material. Zumindest die mit dem PU-Schaumkunststoff in Kontakt kommende Seite der ersten Lage haftet dabei an dem PU-Schaumkunststoff oder geht mit diesem gar eine Verbindung ein. Die andere Seite bildet dann zumindest teilweise die Unterseite der späteren Schuheinlage.

Ist die erste Lage aus einem flexiblen Material gebildet, das für den Schaumkunststoff durchlässig ist, wird erfindungsgemäß vor dem Einlegen der ersten Lage mit dem daran angeformten Kernteil in die erste Schäumformhälfte eines Schäumformwerkzeuges eine zweite flexible Materiallage zwischen die formgebende Oberfläche der Schäumform und der ersten Lage eingelegt, die dann die Unterdecke ausbildet, wobei der Schaumkunststoff die zweite Materiallage bevorzugt nicht durchdringen kann. Die der formgebenden Oberfläche zugewandte Seite der zweiten Lage bildet dann auch hier zumindest teilweise die Unterseite der späteren Schuheinlage.

Hieraus erkennt zumindest der einschlägige Fachmann, dass es im Sinne der Erfindung unerheblich ist, auf welcher Seite der ersten flexiblen Lage das Kernteil angeformt ist. Vom Erfindungsgedanken ist sowohl ein bezüglich der Schuheinlage innenliegendes, orthopädisches Kernteil als auch ein außenliegendes, orthopädisch wirksames Kernteil umfasst. Ist speziell ein Innenliegendes Kernteil gewünscht, so konnte dies im Stand der Technik bisher nur durch vollständige Umschäumung oder durch nachträgliches Bekleben mit einer Unterdecke erreicht werden. Erfindungsgemäß wird dies nun vereinfacht dadurch erreicht, dass das Kernteil in der RIM-Schäumform auf der der formgebenden Oberfläche abgewandten Seite der ersten flexiblen Lage eingelegt und fixiert wird. Nach dem Schäumen des Polsterkörpers bedeckt die erste flexible Lage das Kernteil, so dass es nur noch schemenhaft sichtbar, kann jedoch ertastet werden.

Ist das Kernteil an einer Schaumkunststoff-durchlässigen ersten flexiblen Lage angeformt, kann seine diesbezügliche Orientierung frei gewählt werden, da in diesem Fall eine zweite flexible Lage, die die Unterdecke der späteren Schuheinlage bildet, zwischen der formgebenden Oberfläche der ersten Schäumformhälfte und der ersten flexiblen Lage mit angeformten Kernteil eingelegt und fixiert wird.

Speziell durch das Anschäumen der Unterdecke kann der viel Sorgfalt und Sauberkeit erfordernde Schritt eines nachträglichen Beziehens der Schuheinlage, beispielsweise durch Kleben, zumindest für die Unterseite entfallen. Darüber hinaus erleichtert das Anformen des Kernteils an eine flexible Materiallage, deren Abmessungen gemessen in der Formtrennebene größer sind als die entsprechenden Abmessung der formgebenden Oberfläche der ersten RIM-Schäumformhälfte, nicht nur das Handling des Kernteils, sondern insbesondere dessen Fixierung in einer orthopädisch vorbestimmten Position. Die Fixierung des Kernteils gemäß dem Stand der Technik über Stifte, die in das Kernteil eindringen, oder mittels Presssitz ist diesbezüglich aufwändiger und oftmals ungenauer, speziell beim Einlegen über Presssitz oder beim Einlegen kleiner Kernteile mittels Stiften.

Die Erfindung erreicht mit dem Anformen mittels Spritzgussverfahren des Kernteils an eine flexible Lage, die später in die RIM-Schäumform eingehängt wird, eine genauere Positionierung, da die Abstände der Aufnahmepunkte weiter auseinander liegen, als beispielsweise bei den Haltestiften im Stand der Technik, und so Abweichungen an den Aufnahmepunkten geringeren Einfluss auf die orthopädisch vorbestimmte Lage des Kernteils in der Schuheinlage haben. Zudem kann über das flexible Material der ersten Lage und/oder zweiten Lage eine bessere Abdichtung zwischen den beiden Schäumformhälften erreicht werden, wodurch Nacharbeiten, insbesondere die Entfernung von Schäumfahnen, weitestgehend entfällt. Nach bevorzugt mechanischem Zuschnitt der Schuheinlage(n) ist keine weitere, insbesondere manuelle Nacharbeit an den Schuheinlagen mehr erforderlich.

Mittels der erfindungsgemäß im Spritzgussverfahren hergestellten, orthopädisch wirksamen Kernteile, beispielsweise durch die Verwendung eines PP-, PE-, PS- oder eines TPE-Materiales, kann zudem eine wesentlich geringere Wandstärke des Kernteils erreicht werden, als dies mit Kernteilen aus geschäumten PU möglich ist. Diese erfindungsgemäße Wandstärkenreduzierung ist insbesondere bei der Verwendung der erfindungsgemäßen Schuheinlage in Sportschuhen, Freizeitschuhen, Straßenschuhen, Arbeitsschuhen oder ähnlichem Schuhwerk als orthopädische Schuheinlage vorteilhaft, da die Trageeigenschaften des Schuhs nur minimal beeinflusst werden. Werden darüber hinaus bei der Herstellung des Kernteils beispielsweise faserverstärkte Kunststoffe, wie z.B. CFK- oder GFK-Kunststoffe, also mit Kohlefaser oder Glasfaser verstärkte Kunststoffe, verwendet, so lässt sich eine noch weitere Wandstärkenverringerung des Kernteils erzielen. Die zuvor genannten Spritzguss-geeigneten Kunststoffe, die mit den fachüblichen Kürzel angegeben sind, stellen hierbei nur eine Auswahl spritzgießbarer Materialien dar, auf die die Erfindung jedoch nicht beschränkt ist. Im Sinne der Erfindung können sämtliche thermoplastische als auch thermoelastische Materialien verwendet werden, was auch die Verwendung von TPU (thermoplastisches Polyurethan) umfasst. Die Erfindung umfasst dabei auch duroplastische Werkstoffe als Spritzguss-Werkstoffe, auch wenn deren Einsatz bei Schuheinlagen aufgrund ihrer Sprödigkeit und Bilden scharfer Kanten bei Bruch eher selten vorkommt.

Allgemein gilt, dass je geringer die Wandstärke des Kernteils ausgebildet werden kann, desto dünner kann die Polsterschicht / der Polsterkörper, u.a. auch zum Polstern von den Tragekomfort störenden Kanten ausgebildet werden. Zum Polstern können sowohl weiche als auch harte PU-Schäume verwendet werden, die die gewünschte oder orthopädisch erforderliche Dämpfung bzw. Stabilität der Schuheinlage bereitstellen.

Hinsichtlich der Fußbereiche, über die sich das Kernteil erstreckt, sind erfindungsgemäß keine Beschränkungen vorhanden. So kann sich das Kernteil vom Fersenbereich bis einschließlich dem Zehenbereich erstrecken oder aber auch nur über Teilbereiche, wie beispielsweise den Mittelfußbereich, um insbesondere den Längsgewölbebereich zu unterstützen. Eine Ausbildung des Kernteils, welches beispielsweise den Längsgewölbebereich der Schuheinlage sowie den Fersenbereich umfasst, kann beispielsweise in Teilbereichen freigemacht sein, wie etwa unterhalb des Fersensporns, damit in dem anschließenden PU-Schäumvorgang diese Freimachung mit dämpfendem PU-Schaum gefüllt werden kann, was insbesondere bei Diabetiker-Sohlen bevorzugt ist.

Das erfindungsgemäße Anspritzen/Anformen des Sohlen-stabilisierenden und Fuß-unterstützenden Kernteils an eine erste flexible Lage kann insbesondere auch individuell erfolgen, da die Position des Kernteils im darauf folgenden Schäumvorgang durch die feste Verbindung mit der ersten flexiblen Lage genau beibehalten werden kann. Somit ist es denkbar, das Kernteil schon vor dem Schritt des RIM-Schäumen an die individuellen Bedürfnisse eines Trägers der Schuheinlage angepasst wird. Durch die genaue Übertragbarkeit der Position des Kernteils mittels der ersten Lage, an der es angespritzt/angeformt ist, lässt sich zudem eine hohe und genaue Wiederholbarkeit bei der Positionierung des Kernteils in der erfindungsgemäßen PU-Schaumkunststoff-Schuheinlagen erzielen.

Wie für einen Fachmann leicht ersichtlich, lässt sich mit dem erfindungsgemäßen Herstellverfahren eine große Vielfalt an PU-Schuheinlagen mit einem Sohlen-stabilisierendem Kernteil herstellen, die die unterschiedlichsten Kombinationen von Materialien und/oder Farben und/oder Unterdeckenformen zulassen. Ein Analoges gilt für Oberdecken, wenn eine solche im erfindungsgemäßen RIM-Schäumschritt auf die Oberseite gleich mit angeschäumt wird. Weitere Einzelheiten hierzu später. So können Sohlenhärtevorgaben von Orthopädietechnikern sowohl für das Kernteil als auch für den PU-Schaumkunststoff sowie für die Unterdecke und die Oberdecke individuell und flexibel in einfacher Art und Weise berücksichtigt und umgesetzt werden.

Durch Anspritzen des Kernteils im Spritzgussverfahren an eine Seite einer ersten Lage eines flexiblen Materials, welche beispielsweise die spätere Unterdecke bildet, kann das Kernteil in der späteren Schuheinlage sowohl auf der Innenseite der Schuheinlage als auch auf der Au-ßenseite der Schuheinlage angeordnet sein (siehe oben). Ist das Kernteil auf der Außenseite der späteren Schuheinlage angeordnet, so ist es später für einen Schuhorthopädietechniker zur individuellen Anpassung an den Fuß des Trägers der Schuheinlage leicht zugänglich.

Hinsichtlich des Kernteilmaterials können, insbesondere bei der Anordnung des Kernteils auf der Außenseite der fertigen Schuheinlage, auch Materialien verwendet werden, die mit dem PU-Schaumkunststoff keine feste Verbindung eingehen. Diese Gestaltungsfreiheit kann auch für innenliegende Kernteile verwendet werden, jedoch sollte dann das Kernteil bevorzugt an den freien Seiten von PU-Schaumkunststoff umgeben sein, damit die Position des Kernteils durch den umgebenden PU-Schaumkunststoff und der Unterdecke möglichst gut fixiert ist. Bevorzugt ist hier - bei innenliegendem Kernteil - eine Art Taschenlösung, wobei die Tasche durch die Unterdecke, den Schaumkunststoff und/oder gegebenenfalls durch die Oberdecke ausgebildet bzw. geschlossen wird. Da die Tasche das Kernteil allseitig umgibt, sind die Hafteigenschaften des PU-Schaum-Kunststoffs an dem Kernteil sekundär, weil das Kernteil innerhalb der Schuheinlage in seiner orthopädisch vorbestimmten Lage von der Tasche gehalten wird. Somit können auch Spritzgussmaterialien zum Einsatz kommen, die mit dem PU-Schaumkunststoff keine haftende Verbindung eingehen. Selbstredend auch solche, die gut am PU-Schaumkunststoff anhaften.

Damit die Position des Kernteils beim Herstellen der Schaumkunststoff-Schuheinlage gewährleistet ist, wird das Kernteil erfindungsgemäß an eine Lage eines flexiblen Materials angespritzt, dessen Flächenabmessungen größer sind als die der fertigen PU-Schuheinlage. Die flexible Lage wird dabei bevorzugt, in einer Größe/Länge von einem Rollenmaterial abgelängt, mit der die formgebende Kavität der PU-Schäumform ohne Weiteres abgedeckt werden kann. Bevorzugt erhält man so eine insbesondere rechteckige Form der flexiblen Lage. In einer Ausführungsform werden bevorzugt vor dem Einlegen der so bemessenen Lage Aufnahmelöcher beispielsweise in Randbereiche der Lage gestanzt oder geschnitten, um die Lage beispielsweise an hierfür vorgesehene Stifte in der Spritzgussform aufspannen zu können. Diese Stifte in der Spritzgussform garantieren die Übernahme der Position des Kernteils von der Spritzgussform zur PU-Schäumform, welche erfindungsgemäß Stifte mit denselben Abständen zum Aufspannen der Lage mit dem nun daran angespritzten Kernteil aufweist. Über solche Stifte kann auch die orthopädisch vorbestimmte Position des Kernteils in der späteren Schuheinlage bestimmt werden und genau eingehalten werden.

Die Ausführungsform mit Aufspann-Stiften in den einzelnen formgebenden Werkzeugen stellt dabei nur eine Möglichkeit zum Fixieren einer Lage eines flexiblen Materials in einer (Schäum-)Formhälfte dar. Andere fachübliche Lösungen, wie beispielsweise ein Klemmen, Anheften oder Ansaugen mittels Unterdruck in der Form, sind dabei vom Erfindungsgedanken ebenfalls umfasst. Wichtig ist dabei nur, dass die orthopädisch vorbestimmte relative Lage des Kernteils zum Polsterkörper in der PU-Schäumform eindeutig eingehalten werden kann, d.h. dass die Lage mit dem angespritzten Kernteil in eine genau vorbestimmte orthopädisch wirksame Position in eine erste Schäumformhälfte eingelegt und dort fixiert werden kann. In einer ersten Ausführungsform haben sich hier sowohl in der Spritzgussform und in der jeweiligen Schäumformhälfte analog beabstandet angeordnete Aufspannstifte für das Aufspannen der ersten Lage als geeignet erwiesen, wobei jede andere Art eines positionsgenauen Einlegens und Fixieren der ersten Lage vom Erfindungsgedanken umfasst ist, z. B. Einlegen mittels Schablonen und Fixieren mit Klemmschienen oder Unterdruck.

In einer weiteren bevorzugten Ausführungsform der Erfindung sind die Aufspannstifte in den jeweiligen Formhälften - zum Spritzgießen des Kernteils und/oder für das Anschäumen von PU an die Lage mit dem angespritzten Kernteil - senkrecht zur jeweiligen Schließrichtung des Formwerkzeugs beweglich, um einerseits Verformungen des Materials der Lage zum Ausbilden eines in allen drei Dimensionen geformten Kernteils und der späteren Schaumkunststoff-Schuheinlage zu ermöglichen und andererseits einer Beschädigung des Materials der flexiblen Lagen entgegen zu wirken. Weiter bevorzugt ist diese Beweglichkeit der Stifte durch elastische Kräfte gedämpft, sodass das Material der Lage durch die elastisch gelagerten Stifte senkrecht zur Schließrichtung über der jeweiligen Formhälfte (Formnest) gespannt wird. Dadurch kann einerseits gewährleistet werden, dass die Decke faltenfrei über dem jeweiligen Formnest gespannt ist, um Produktionsfehler zu vermeiden, und andererseits wird eine gewisse Nachgiebigkeit während des formgebenden Schrittes zugelassen, insbesondere wenn dreidimensional gewölbte Formteile/Kernteile an das Material der ersten Lage angespritzt/angeschäumt werden. Eine starre Aufspannung des Lagen-Materials der Decke könnte hier zu Falten und im Extremfall auch zu Rissen im Material führen, insbesondere in der Nähe der Aufspannstellen.

In einer anderen Ausgestaltung der Erfindung ist es ebenfalls vorstellbar, dass die Randbereiche der Lage ortsfest bezüglich der jeweiligen formgebenden Oberfläche des Formwerkzeuges fixiert sind und das verwendete Lagenmaterial für die Unterdecke selbst ausreichend elastische Eigenschaften aufweist, um Verformungen/Verwölbungen während des Spritzgießens oder RIM-Schäumens elastisch kompensieren zu können.

Egal welche Ausgestaltung verwendet wird, es sollte darauf geachtet werden, dass insbesondere, bei dreidimensionaler Ausgestaltung des Kernteils - was bei den vielen Ausführungen der Fall sein wird - eine gewisse dreidimensionale (elastische) Nachgiebigkeit der fixierten Lage(n) gegeben ist, damit ein optimales Herstellverfahren mit einer hohen Produktqualität erreicht wird. Dies gilt beispielsweise auch für die Aufspannung der flexiblen Lage mit angespritztem Kernteil in der RIM-Schäumform, wobei das Kernteil in den meisten Fällen zwar eine sehr geringe Wandstärke aufweist, jedoch die flexible Lage im angespritzten Bereich aufgrund seiner orthopädischen Funktion versteift.

Wie der Fachmann aus den obigen Erläuterungen bereits entnommen hat, können alle gängigen Materialien, als auch nicht gängigen Materialien als Material für die erste flexible Lage oder das Unterdeckenmaterial verwendet werden, so lange zumindest an einer Seite der ersten flexiblen Lage das Material des angespritzten Kunststoffs des Kernteils daran anhaftet und sich die Innenseite der ersten flexiblen Lage mit dem PU-Schaumkunststoff verbindet bzw. der Schaumkunststoff daran anhaftet. Dabei sind auch Materialien für die flexible Lage geeignet, die gegebenenfalls für den flüssigen Spritzguss-Kunststoff durchlässig sind. In diesem Fall ist erfindungsgemäß eine zweite flexible Lage vorgesehen, die dann bevorzugt PU-Schaumkunststoff-dicht ist und die Unterdecke der Schuheinlage ausbildet. Dem Fachmann sind insofern keine Grenzen gesetzt. Bei der Wahl des Materials ist er lediglich hinsichtlich der Anforderungen an die geforderten, vor allem orthopädischen, Eigenschaften der fertigen PU-Schaumkunststoff-Schuheinlage mit Einlagen-stabilisierendem Kernteil gebunden. Hier kann weiter insbesondere an Eigenschaften, wie mechanische und thermische Stabilität, Tragekomfort, Feuchtigkeitsaufnahme, Abriebfestigkeit, Rutschfestigkeit, Nachbearbeitbarkeit durch einen Schuhorthopädie-Techniker und dergleichen gedacht werden.

Nach dem Anformen (Spritzgießen) des Kernteils in einem Teilbereich der flexiblen Lage kann diese aus der ersten flexiblen Lage und dem angeformten Kernteil bestehende Baugruppe aus der Spritzgussform entnommen werden, beispielsweise durch Aushängen, d.h. Lösen der flexiblen Lage von den Positionier-Hilfen. In diesem nächsten Verfahrensschritt kann das Kernteil und/oder das die Unterdecke bildende Material individuell an den Träger der Schuheinlage angepasst oder behandelt werden, bevor die Lage mit dem daran angeformten Kernteil dem RIM-Schäumverfahren zugeleitet wird. Hier kann beispielsweise an das Aufbringen einer haftvermittelnden Schicht gedacht werden.

Die flexible Lage mit dem angeformten Kernteil wird dann zum Schäumen im RIM-Verfahren mit denselben Fixierpunkten bzw. mit denselben Durchlässen bzw. Löchern, die im Spritzgusswerkzeug zum Fixieren verwendet wurden, in eine erste Schäumformhälfte des RIM-Schäumwerkzeuges eingelegt und dort fixiert. Bevorzugt, wie oben erläutert, derart, dass die Position des Kernteils im Schäumwerkzeug der orthopädisch vorbestimmten Position in der Schuheinlage entspricht. Zum Ausbilden der (fertigen) Schuheinlage werden dann die den Schaumkunststoff bildenden Komponenten in die Schäumform eingebracht und können in der geschlossenen Schäumform aufquellen und so die Kavität der Schäumform unter gleichzeitiger Ausbildung der Schuheinlage mit einem orthopädischen Fußbett auf der Oberseite ausschäumen. Hierbei werden üblicherweise ein Polyol und ein Isocyanat verwendet, welche durch Kontakt miteinander unter Bildung von Wasserstoff exotherm zu einem PU-Schaumkunststoff reagieren. Da diese Reaktion dem hierfür einschlägigen Fachmann bekannt ist, wird auf weitere Erläuterungen diesbezüglich verzichtet.

Beim Ausschäumen der RIM-Schäumform werden zumindest Teilbereiche der Innenseite der Materiallage mit Schaumkunststoff angeschäumt und verbinden sich mit dem Schaumkunststoff oder haften daran an. Wie oben erläutert kann das an die erste Materiallage angeformte Kernteil entweder auf der Innenseite angeordnet sein, sodass es ebenfalls mit Schaumkunststoff angeschäumt wird oder ist auf der künftigen Außenseite der Unterdecke angeordnet, so dass zumindest seine Außenseite nicht mit Schaumkunststoff angeschäumt wird. Auch wenn das Kernteil an der Innenseite der Unterdecke angeordnet ist, so ist es nicht zwingend erforderlich, dass die mit dem Schaumkunststoff in Berührung kommende Innenseite des Kernteils am Schaumkunststoff haftet, bzw. kleben bleibt. Die Position des Kernteils in der Schuheinlage wird erfindungsgemäß auch in diesem Fall durch die Verbindung des Kernteils mittels der ersten Lage bzw. der Innenseite der Unterdecke festgelegt. Das Kernteil wird so in Art einer Tasche, die aus Schaumkunststoff, erster Lage und/oder Unterdecke gebildet wird, festgehalten.

In einer bevorzugten Ausführungsform ist die erste flexible Materiallage Schaumkunststoff-dicht, insbesondere für den nicht ausgehärteten Schaumkunststoff, und dient dann gleichzeitig als Materiallage für die Ausbildung / Anbindung der Unterdecke. In einer solcher Ausführungsform ist die Materiallage für eine solche Unterdecke weiter bevorzugt beispielsweise zweilagig, wobei die Innenseite durch eine Schicht gebildet wird, die sich gut mit dem Schaumkunststoff verbindet oder gut daran anhaftet bzw. daran kleben bleibt, oder in die der Schaumkunststoff eindringen, aber nicht durchdringen kann, da die zweite Schicht, die die Unterseite der Schuheinlage bildet, als Schaumkunststoff-Sperrschicht ausgebildet ist. So kann für die zweite, die äußere Schicht jedes andere Material gewählt werden, ohne Rücksicht darauf, ob es sich gut oder schlecht mit dem Schaumkunststoff verbindet. Solche zweilagigen Materialien sind im Stand der Technik hinlänglich bekannt und bedürfen daher an dieser Stelle keiner weiteren Ausführung. Für die Außenseite der Unterdecke werden jedoch Materialien bevorzugt, die der Schuheinlage rutschfeste Eigenschaften verleihen, da dadurch die orthopädischen Eigenschaften und der Tragekomfort der Schuheinlage besser gewährleistet und somit verbessert werden kann. Hierbei kann als Sperrschicht ein Material verwendet werden, das mit dem verwendeten Schaumkunststoff keine Verbindung eingeht, insbesondere keine klebende oder haftende Verbindung, wie z.B. ein Elastomer oder ein gummiartiges Material. Über die auf der Innenseite den nicht ausgehärteten Schaumkunststoff aufnehmende faserhaltige Schicht wird dann mittels des Schaumkunststoffs die feste Verbindung der Materiallage mit dem Schaumkunststoff erreicht. Gleichzeitig kann in dieser Ausführungsform das Material der zwei- oder mehrschichtigen Unterdecke durch das Eindringen des Schaumkunststoff in die faserhaltige Schicht verstärkt bzw. versteift werden. Dieser Effekt tritt dadurch auf, dass der flüssige/breiige Schaumkunststoff zwar zwischen die Fasern eindringen kann, bzw. von diesem aufgesogen wird, aber innerhalb, beispielsweise des Textils, nicht so stark unter Bildung von Zellen/Poren aufquellen / expandieren kann, weil dies durch das Textil verhindert wird. Somit weist der Schaumkunststoff in der textilen Schicht eine größere Dichte auf. Gleichzeitig behindern die Fasern die Beweglichkeit/Elastizität des Schaumkunststoffs und der Fasern in Faserrichtung und quer zur Faserrichtung, sodass eine insgesamt transversale Versteifung einer solchen zweischichtigen Materiallage erreicht wird. Jedoch bleibt die Dämpfungswirkung in Dickenrichtung der Materiallage und der Schuheinlage nahezu unverändert, wodurch erfindungsgemäß eine dünne, jedoch sehr formstabile Schaumkunststoff-Schuheinlage mit hohen Dämpfungseigenschaften und geringer Verformungsneigung herstellbar ist.

Der Fachmann erkennt aus den obigen Ausführungen, dass es dabei unerheblich ist, ob das Kernteil an die Außenseite oder die Innenseite einer solchen zwei- oder mehrschichtigen Materiallage angeformt ist oder ob eine solche zwei- oder mehrschichtige Materiallage ohne angeformtes Kernteil beispielsweise als Material für eine Oberdecke verwendet wird. Der Fachmann erkennt auch, dass wenn das Kernteil auf der Seite angeformt werden soll, auf der die Fasern vorhanden sind, eine Haftverbindung des Spritzgussmaterials mit dem Material der Schaumkunststoff-(Sperr-)Schicht ebenfalls nicht zwingend erforderlich ist, weil die faserhaltige Schicht der zwei- oder mehrschichtigen Materiallage das im Spritzgussverfahren angeformte Kernteil festhält. Somit kann die Verwendung einer zwei- oder mehrschichtigen Materiallage ein größeres Spektrum an verwendbaren Materialien zulassen, als dies beispielsweise für einschichtige Materialien der Fall ist.

Dabei umfassen zwei- oder mehrschichtige Materialien auch Ausgestaltungen, bei denen eine Schicht mit Ausnehmungen / Freimachungen versehen ist, durch die das Material einer anderen Schicht sichtbar ist. Hiermit lassen sich neben optischen Effekten auch orthopädische Effekte, insbesondere unterschiedliche Härtebereiche in verschieden Fußaufstandsbereichen erzielen. In einer weiteren bevorzugten Ausführungsform können so Ausnehmungen in einer Materialschicht im Schäumwerkzeug - sowohl in der ersten als in der zweite Schäumformhälfte - angeordnet werden, dass die Ausnehmungen während des RIM-Schäumens mit Schaumkunststoff gefüllt werden. Dabei verhindert eine bevorzugt transparente Außenschicht ein Überschäumen außerhalb der Ausnehmungen (Fenster). Hiermit können insbesondere die Bereiche des Fersensporns, der Zehengrundgelenke oder Zehen selbst weicher ausgestaltet werden, als in Bereichen ohne diese Ausnehmungen, da dort das Material der Unterdecke die Schuheinlage versteift. In einem bevorzugten Ausführungsbeispiel wird als transparente Schicht beispielsweise eine dünne PU-Folie verwendet, die nahezu nicht-sichtbar die Freimachungen begrenzt und dem PU-Schaumkunststoff oder dem Material der ersten flexiblen Lage, das in diesen freigemachten Bereichen sichtbar ist, ein hochwertiges Erscheinungsbild verleiht.

Bevor die Komponenten zum Erzeugen des PU-Schaumkunststoffs in die Schäumform eingebracht werden, kann in einer weiteren Ausführungsform der Erfindung auf die andere, die zweite Schäumformhälfte eine weitere Lage eines flexiblen Materials gelegt oder aufgespannt werden, welche dann die Oberdecke bildet. Durch Einlegen einer weiteren Lage auf die zweite Formhälfte der Schäumform, welche die Oberdecke bildet, kann insbesondere ein weiterer Klebeschritt an der erfindungsgemäß ausgebildeten Schuheinlage vermieden werden, nämlich der des Aufbringens einer Oberdecke - beispielsweise durch Aufkleben oder Aufbügeln. Durch Einlegen einer weiteren zweiten bzw. dritten Lage in die zweite Schäumformhälfte vor dem Einbringen der den Schaumkunststoff erzeugenden Komponenten in die Schäumform können die jeweiligen Innenseiten der Unterdecke und der Oberdecke mittels des sich bildenden Schaumkunststoffs ohne weiteres miteinander verbunden werden. Dabei kann vor dem Einlegen der Materiallage für die Oberdecke an dessen Innenseite eine weitere orthopädische Komponente, beispielsweise eine Pelotte oder ähnliches, befestigt sein. Beim Einlegen und Fixieren der Materiallage für die Oberdecke wird bevorzugt, analog zum Vorgehen bei der Positionierung der Materiallage der Unterdecke, diese so in die zweite Schäumformhälfte der RIM-Schäumform eingelegt und fixiert, dass weitere orthopädische Komponenten an orthopädisch vordefinierten Lagen zum Liegen kommen.

Weiter vorstellbar ist auch, dass sowohl an der Lage des Materials für die Unterdecke als auch an die Lage des Materials für die Oberdecke jeweils ein Kernteil/Formteil angeformt ist und beide Lagen entsprechend der vorbestimmten orthopädischen Position auf die jeweilige Formhälfte des Schäumwerkzeuges aufgespannt werden.

Nachdem zumindest eine der Materiallagen, d.h. die der ersten flexiblen Lage und optional die der Unterdecke sowie ggf. die der Oberdecke, auf ihre jeweiligen Formhälften aufgespannt wurden, wird das Schaumkunststoffausgangsmaterial, d.h. die Komponenten zum Erzeugen des PU-Schaumkunststoffs derart zwischen die Innenseiten der beiden Lagen eingebracht, dass sich die beiden Innenseiten über den sich in der geschlossenen Schäumform bildenden Schaumkunststoff verbinden können.

Nachdem die Komponenten zum Erzeugen des PU-Schaumkunststoffs in die Schäumform eingebracht und zur Reaktion gebracht wurden, kann der Schaumkunststoff, wie oben bereits beschrieben, die RIM-Schäumform im geschlossenen Zustand durch chemische Reaktion der beiden Komponenten ausschäumen, bzw. der Schaumkunststoff kann innerhalb der Kavität der Schäumform quellen und expandieren.

Nachdem die Schäumform vollständig ausgeschäumt ist und der durch die chemische Reaktion gebildete PU-Schaumkunststoff ausgehärtet ist, kann die Schäumform geöffnet werden und die PU-Schuheinlage-Rohlinge aus der RIM-Schäumform entnommen werden. In diesem Zustand ist zumindest die flexible Lage der Unterdecke fest mit dem Schaumkunststoff verbunden und auf der Oberseite der späteren Schuheinlage ist gemäß der zweiten Schäumformhälfte ein orthopädisches Fußbett ausgebildet, das gegebenenfalls erfindungsgemäß gleich mit einer Oberdecke bezogen ist. Wurde vor dem Schließen der RIM-Schäumform in die zweite Schäumformhälfte eine weitere Lage eines flexiblen Material eingelegt und fixiert, sind nach Aushärten des Schaumkunststoff in der Schäumform die beiden flexiblen Lagen - die für die Oberdecke und die für die Unterdecke - über ihre jeweiligen Innenseiten miteinander verbunden.

Da die Lage/ die Lagen aufgrund ihrer erforderlichen Fixierung in den einzelnen Formhälften der jeweiligen Formwerkzeuge größer gewählt wurden als die Abmessungen der Schuheinlage, muss der Schaumkunststoff-Schuheinlagen-Rohling nach dem Entnehmen aus der RIM-Schäumform in seiner Kontur geeignet zugeschnitten werden. Dies kann beispielsweise durch manuelles Schneiden, bevorzugt jedoch durch maschinelles Stanzen erfolgen. Nach dem Zuschnitt des Schuheinlagen-Rohlings zum Ausbilden der Schuheinlage ist diese, falls sie von einem Schuhorthopädietechniker nicht nachgearbeitet werden soll, gebrauchsfertig.

Wie bereits oben für das Ausführungsbeispiel mit angeschäumter Unterdecke erläutert, können je nach Einsatzzweck und auch gewünschtem Erscheinungsbild entweder die Lagen aus dem flexiblen Material der ersten Lage, der der Unterdecke als auch die Lagen aus dem flexiblen Material der Oberdecke oder alle Lagen so gestaltet sein, dass sie für den PU-Schaumkunststoff durchlässig oder undurchlässig sind. Als Beispiel für die Unterdecke wurde oben bereits eine rutschfeste Außenseite für die Unterseite der Schuheinlage angegeben. Für die Oberdecke wird man allerdings eine den Tragekomfort verbessernde Außenseite - die die Oberseite der Schuheinlage bildet - bevorzugen, die beispielsweise weich ist und/oder den Fuß des Trägers der Schuheinlage wärmt und/oder optisch ansprechend ist. Je nach verwendetem Material, z.B. bei einem Vlies, ist daher ggf. eine PU-Schaumkunststoff-Sperrschicht für die Innenseite vorzusehen, damit der im Schäumvorgang erzeugte Schaumkunststoff nicht an die Außenseite gelangt.

Ggf. muss auch hinsichtlich des Spritzgussprozesses zum Ausbilden des Kernteils eine für das Spritzgussmaterial, insbesondere das flüssige Spritzgussmaterial undurchlässige Sperrschicht an der Materiallage vorgesehen werden. Soll beispielsweise ein Kernteil an die Außenseite der Unterdecke angeformt werden und haftet der PU-Schaum an dem verwendeten Spritzgussmaterial nur schlecht oder gar nicht, so kann das Material der Unterdecke zumindest einseitig entweder auf seiner Außenseite oder seiner Innenseite mit einer Sperrschicht versehen sein, durch die der Spritzgusskunststoff in seinem flüssigen Zustand nicht hindurchtreten kann. Damit kann gewährleistet werden, dass die Innenseite der Unterdecke vollflächig über den Schaumkunststoff verbunden werden und keine losen Bereiche, insbesondere im Bereich des außenliegenden Kernteils verbleiben, in denen sich das Polstermaterial (Schaumkunststoff) oder das der Oberdecke aufwerfen und Falten bilden kann. Selbstredend sollte die Innenseite der Unterdecke ein Material sein, bzw. aufweisen, welches sich mit PU-Schaum verbinden lässt.

Ist das Material der Oberdecke oder der Unterdecke beispielsweise ein Material, welches sich im Allgemeinen schlecht mit PU-Schaumkunststoff verbindet, so sollte auf die jeweilige Innenseite eine (zusätzliche) Schicht aufgetragen werden, welche sich gut mit dem PU-Schaumkunststoff verbindet. Dies kann beispielsweise eine Kunststoff-, insbesondere PU-Schicht sein oder ein Textil oder ein Vlies, welches auf die Innenseite des Lagenmaterials beispielsweise aufgeklebt ist. Somit kann der Schaumkunststoff beispielsweise in das Textil - ein Gewebe oder ein Vlies - eindringen und dadurch die Unterdecke oder Oberdecke gegenüber der jeweiligen anderen Ober- oder Unterdecke halten.

In weiteren Ausführungsformen kann zusätzlich zu den Materiallagen für die Ober- bzw. Unterdecke eine (zusätzliche) Verstärkungslage eingebracht werden, die den Schaumkunststoff zwischen der Oberdecke und der Unterdecke transversal stabilisiert. In einer Ausführungsform wird eine solche Verstärkungslage beispielsweise bevorzugt während des Spritzgießens des Kernteils an die erste Lage mittels des Spritzgusskunststoffes sozusagen an die erste Lage mit angeheftet. Dabei muss das Material für die Verstärkungslage nicht Spritzkunststoff-dicht sein, sondern kann wiederum ein Textil beispielsweise ein Gewebe, Gewirk oder ein Vlies oder ein ähnliches faserhaltiges Material sein, welches bevorzugt durch den in der Schäumform erzeugbaren PU-Schaumkunststoff durchdringbar ist. Damit werden die Fasern dieser Zwischenlage von Schaumkunststoff ummantelt und bilden mit diesem sozusagen einen faserverstärkten Schaumkunststoff aus, der eine Verformung des Schaumkunststoffes der Schuheinlage in Querrichtungen, also in deren Breitenrichtung oder Längenrichtungen (transversal), be- bzw. verhindert, jedoch die Dämpfungswirkung des PU-Schaumkunststoffes in Dickenrichtung - in vertikaler Richtung bei Gebrauch der Schuheinlage - nahezu unbeeinflusst lässt.

In einer weiteren Ausführungsform kann eine solche Verstärkungslage auch erst vor dem Schäumvorgang entweder auf die Innenseite der ersten Lage oder auf die Innenseite der zweiten oder dritten Lage aufgebracht werden und entweder in der ersten Schäumformhälfte oder in der zweiten Schäumformhälfte fixiert werden.

Üblicherweise werden die Formnester der einzelnen Formhälften der Formwerkzeuge vor dem eigentlichen Formvorgang mit einem Trennmittel benetzt, damit die Ausformung des Formteils, hier entweder des Kernteils oder der Schuheinlage, erleichtert bzw. ein Ankleben der jeweiligen Lagen/Bauteile an das Formwerkzeug vermieden wird. Weiter können die jeweiligen Außenseiten der Materiallagen für die Oberdecke und die Unterdecke vor dem Einlegen in die jeweilige Formhälfte zuvor mit einer entsprechenden Schutzschicht versehen werden, damit das Erscheinungsbild verschlechternde Rückstände des Trennmittels vermieden werden. Eine PU-Schaumkunststoff-undurchlässige und nicht am PU-Schaumkunststoff haftende Schutzschicht auf der Außenseite der Unter- und/oder Oberdecke ist jedoch bevorzugt vor dem RIM-Schäumen in die entsprechende Schäumform einzulegen, wenn im Lagenmaterial sogenannte Fenster, d.h. Öffnungen oder Freimachungen, vorgesehen sind, in der Schaumkunststoff während der Expansion in der Schäumform eindringen soll, aber an der fertigen Schuheinlage nicht mit dem eigentlichen Deckenmaterial bedeckt sein soll. Mit solchen Fenstern in der Ober- und/oder Unterdecke können empfindliche Bereiche für den Fuß des Trägers der erfindungsgemäßen Schuheinlage, wie beispielsweise der Bereich der Zehengrundgelenke, weicher ausgestaltet werden als Bereiche, die mit einer Decke versehen sind.

Auch wenn obige Ausführungsformen der Einfachheit halber zum Erläutern des erfindungsgemäßen Verfahrens und der erfindungsgemäßen Schuheinlage am Beispiel der Herstellung für eine einzelne Schuheinlage beschrieben wurden, so erkennt der einschlägige Fachmann, dass Schuheinlagen bevorzugt paarweise hergestellt werden und somit die Formnester in den Formwerkzeugen auch paarweise angeordnet sein können. Selbstredend sind vom Erfindungsgedanken daher auch Formwerkzeuge umfasst, welche ein oder auch mehr als ein Paar Kernteile bzw. Schuheinlagen in einem Formgebungszyklus ausbilden können. So sind Doppel-, Dreifach-, Vierfachwerkzeuge, etc., welche zwei, drei, vier, etc., Paar an eine flexible Lage angeformte Kernteile bzw. Paare von Schaumkunststoff-Schuheinlagen in einem Formschritt gleichzeitig ausbilden können, ohne Weiteres von einem Fachmann im Bereich des Möglichen.

Mit dem erfindungsgemäßen Verfahren zum Herstellen einer orthopädischen PU-Schaumkunststoff-Schuheinlage mit einem im RIM-Schäumverfahren auf der Oberseite erzeugten orthopädischen Fußbett, einer direkt angeschäumten Unterdecke sowie mit einem in einem vorangegangenen Prozessschritt an eine erste Lage mittels Spritzgießen angespritzten Kernteils, können folglich in einem weitestgehend automatisierten Verfahren Schaumkunststoff-Schuheinlagen hergestellt werden, bei deren Herstellung insbesondere ein Klebeschritt oder sogar mehrere Klebeschritte zum Aufbringen einer oder mehrerer Decken auf die erfindungsgemäße Schuheinlage vermieden werden. Somit wird zumindest ein sehr viel Sorgfalt und Sauberkeit erfordernder Prozessschritt des Aufbringens, meist mittels Klebens oder Aufbügeln einer Decke, der zudem üblicherweise hohe Kosten verursacht, vermieden. Nach Durchführung des erfindungsgemäßen Verfahrens steht eine gebrauchsfertige, jedoch gegebenenfalls durch einen Schuhorthopädietechniker individuelle anpassbare Schuheinlage bereit, die nicht mehr durch den Schuheinlagen-Hersteller nachbearbeitet werden muss, sobald deren Zuschnitt erfolgt ist.

Die Nacharbeit bezieht sich hierbei auf eine Nacharbeit beim Hersteller von Schuheinlagen. Die Anpassarbeit der erfindungsgemäßen PU-Schaumkunststoff-Schuheinlage durch einen Schuhorthopädietechniker soll hierbei nicht unter den Begriff Nacharbeit fallen, da die gemäß der Erfindung hergestellten Sohlen, gegebenenfalls nach Aufbringen einer zusätzlichen Oberdecke gebrauchsfertig für den Endkunden sind, beispielsweise zum Einlegen in Schuhe, die kein Fußbett aufweisen.

Bei den durch das erfindungsgemäße Verfahren hergestellten orthopädischen Schuheinlagen ist es dabei nicht zwingend erforderlich, dass die Unterdecke die komplette Unterseite der Schuheinlage überdeckt, da im Allgemeinen der verwendete PU-Schaumstoff ausreichend resistent ist, um direkt in ein Inneres eines Schuhs eingelegt werden zu können. Somit kann beispielsweise der Vorderfußbereich, insbesondere der Zehenbereich, wesentlich dünner ausgebildet werden, als dies mit herkömmlichen Verfahren möglich war. Es ist dabei auch vorstellbar, dass der Vorderfußbereich und/oder der Zehenbereich lediglich aus der Oberdecke besteht und eine Polsterung durch PU-Schaumstoff nur im Mittenfußbereich und im Fersenbereich oder allein im Fersenbereich erfolgt. Dies hängt von den orthopädischen Anforderungen an die Schuheinlage ab.

Genauso hängt die Größe und Form des spritzgegossenen Kernteils von den orthopädischen Anforderungen ab, die an die erfindungsgemäße Schaumkunststoff-Schuheinlage gestellt werden. So kann das Kernteil die gesamte Außenseite/Unterseite der Schuheinlage bilden. Es ist jedoch auch vorstellbar, dass das Kernteil nur in Teilbereichen der Unterseite der Schuheinlage sichtbar ist. Weiterhin, wie sich aus den obigen Erläuterungen ohne Weiteres ergibt, kann das Kernteil, auch wenn es nur in Teilbereichen der Schuheinlage ausgebildet ist, komplett von der Unterdecke überzogen sein, womit sich seine Anwesenheit zwar ertasten lässt, jedoch nicht unbedingt auf den ersten Blick erkennbar ist. Auch eine nachträglich individuelle direkte Anpassbarkeit des Kernteils an den Fuß des Trägers wäre hier nicht gegeben.

Wie oben bereits angedeutet, kann das Material der Oberdecke als auch das Material der Unterdecke aus verschiedensten, aber auch gleichen Materialien bestehen, bzw. es können Kombinationen von verschiedenartigsten Materialien zum Einsatz kommen, beispielhaft sei hier als Materialauswahl Leder, Kunstleder, Alcantara, Textil, Vlies, Kunststoff, Kautschuk oder ähnliches genannt, wobei die Aufzählung nicht vollständig sein kann. Gerade zur Erzielung ästhetischer Aspekte oder orthopädisch sekundärer Eigenschaften, wie Rutschfestigkeit, Wärmedämmung, elektrische Isolation, Durchdrückfestigkeit, Feuchtigkeitsaufnahme, etc. lassen den Einsatz verschiedenster Materialien zu, die bevorzugt in bahnartigen Lagen oder auch als Platten für die Verwendung als Oberdecke oder Unterdecke zum Einsatz kommen können.

So ist eine Verwendung von Metallfolien als Wärmeschutz oder auch Ableitung statischer Aufladung ebenso vorstellbar, wie z.B. eine dünne Teflonschicht zum Bereitstellen eines Durchdrückschutzes gegen spitze Gegenstände. Dem Fachmann sind insoweit keine Grenzen hierfür gesetzt, so lange die entsprechende Lage/Bahn zum Anformen eines spritzgegossenen Kernteils geeignet ist und/oder geeignet ist, um mit dem PU-Schaumkunststoff, der in einer RIM-Schäumform erzeugt wird, dauerhaft mit der Innenseite der zumindest einen Lage für die Unterdecke oder dauerhaft mit den Innenseiten der Materiallagen für die Unterdecke und die Oberdecke verbunden zu werden.

Für das erfindungsgemäße Verfahren sind, wie oben bereits angedeutet, die Formwerkzeuge zum Herstellen der erfindungsgemäßen orthopädischen Schuheinlagen insofern anzupassen, als dass das in einem ersten Schritt durch Spritzguss an eine erste flexible Lage angeformte Kernteil, welches durch das Material der ersten Lage gehalten wird, in der entsprechenden Schäumformhälfte der RIM-Schäumform in der orthopädisch vorbestimmten Lage zum Liegen kommt. Hierbei können verschiedene Vorrichtungen an den Formwerkzeugen zum Einsatz kommen, wie beispielsweise Aufnahmestifte zum Aufnehmen der entsprechenden flexiblen Lagen über Löcher in den flexiblen Lagen, aber auch Fixierleisten oder Klemmvorrichtungen, die eine Übertragung der Position des Kernteils relativ zur flexiblen Lage, an die es angespritzt ist, auf die RIM-Schäumform zulassen.

Zumindest in den beiden Schäumformhälften der RIM-Schäumform sind die jeweiligen Fixiereinrichtungen der jeweiligen Lagen so anzuordnen, dass diese beim Schließen der beiden Schäumformhälften zum Ausbilden einer Kavität nicht kollidieren. Werden beispielsweise Stifte zum Einhängen/Aufspannen und Fixieren der flexiblen Lagen in den Schäumformhälften verwendet, ist sicherzustellen, dass die Stifte der einen Formhälfte in Bohrungen in der anderen Formhälfte eintreten, damit es mit den Stiften der jeweils anderen Formhälfte nicht zu Kollisionen kommt. Vorstellbar hier ist auch ein konzentrisches Ineinandergreifen der jeweiligen Halte-/Aufspannstifte für die flexiblen Lagen derart, dass jeweiligen Lagen vor dem Einlegen in irgendeine der Formhälften - d.h. auch in eine der Formhälften des Spritzgusswerkzeuges - mit einem einzigen Lochbild beispielsweise mit nur einem Stanzwerkzeug vorgenommen werden können. Dieses Stanzbild für die Aufnahme der jeweiligen Lagen in den Formwerkzeugen kann bevorzugt auch für das Ausstanzen der Kontur der Schuheinlage übernommen werden, womit eine Aufsummierung von Lagetoleranzen weitestgehend unterbunden werden kann.

Im Folgenden wird für beispielhafte Ausführungsformen das oben beschriebene erfindungsgemäße Verfahren zum Herstellen erfindungsgemäßer orthopädischer Schaumkunststoff-Schuheinlagen mit einem die Schuheinlage stabilisierenden spritzgegossenen Kernteil, mit einem auf der Oberseite im RIM-Schäumverfahren ausgebildeten Fußbett sowie mit einer über den in einer RIM-Schäumform erzeugten PU-Schaumkunststoff verbundenen Unterdecke, anhand von Figuren detaillierter dargestellt und beschrieben, wobei die beispielhaft gezeigten Ausführungsformen den Erfindungsgedanken nicht limitieren. Es zeigen:
- Figur 1: schematisch zwei Formhälften eines Spritzgusswerkzeuges gemäß der Erfindung, welche nebeneinander angeordnet sind;
- Figur 2: schematisch eine Formhälfte der Figur 1 mit aufgespannter erster Lage;
- Figur 3: schematisch eine erste Lage mit angeformten spritzgegossenem Kernteil sowie mit einer Verstärkungslage;
- Figur 4: schematisch zwei Formhälften einer RIM-Schäumform gemäß einer ersten Ausführungsform der Erfindung mit davor angeordneter erster und zweiter Lage;
- Figur 5: schematisch zwei Formhälften einer RIM-Schäumform gemäß einer zweiten Ausführungsform der Erfindung mit davor angeordneter erster Lage mit Verstärkungslage und einer Oberdecke;
- Figur 6: schematisch einen mit dem erfindungsgemäßen Verfahren hergestellten Schuheinlagen-Rohling mit außen liegendem Kernteil.

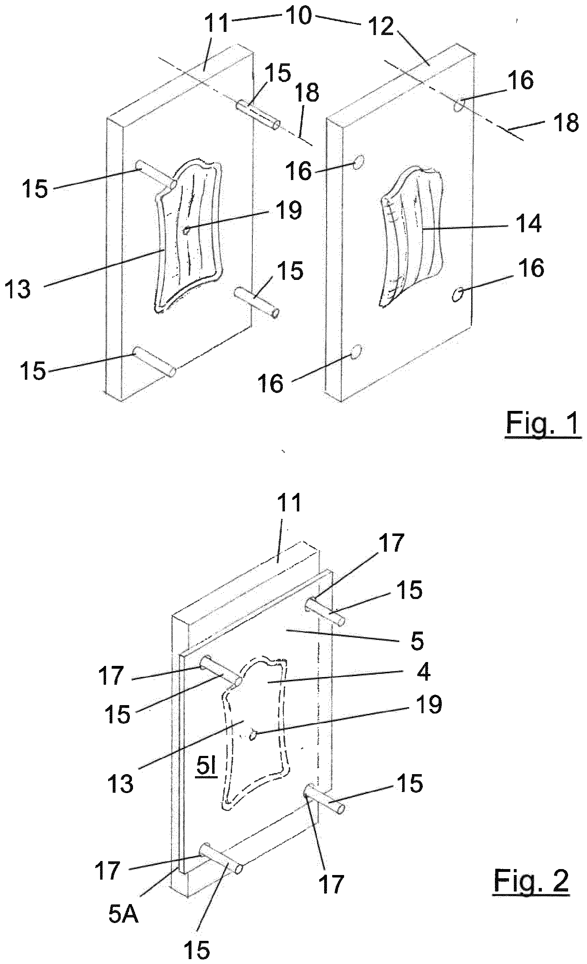

Figur 1 zeigt schematisch in einer perspektivischen Darstellung zwei Formhälften 11, 12 eines Spritzgusswerkzeuges 10, wobei die beiden Formhälften 11,12 zur besseren Erkennbarkeit von Einzelheiten nebeneinander dargestellt sind, und nicht in Schließrichtung 18 des Spritzgusswerkzeuges 10. Beispielhaft ist die erste Formhälfte 11 als Anspritzseite ausgebildet und zeigt eine erste beispielsweise konkav ausgebildete formgebende Oberfläche 13, welche beispielsweise Teilbereiche der Unterseite 9 der herzustellenden Schaumkunststoff-Schuheinlage 1 bildet, falls im Spritzgussschritt des erfindungsgemäßen Verfahrens ein Kernteil 4 an die Außenseite 5A einer ersten Lage 5 (vgl. Figur 2) angespritzt wird. Die Gegenseite, d.h. die formgebende Oberfläche 14 der zweiten Formhälfte 12 ist entsprechend beispielsweise konvex ausgebildet und bildet zusammen mit der ersten formgebenden Oberfläche 13 im geschlossenen Zustand des Spritzgusswerkzeuges 10 eine Kavität, die über den Anspritzpunkt 19 gefüllt werden kann. Die erste Formhälfte 11 weist ferner Aufspannstifte 15 auf, auf die die erste Lage 5 mittels darin ausgebildeten Aufspannlöchern 17 faltenfrei eingehängt/aufgespannt werden kann. Beim Schließen der Spritzgussform 10 in Richtung der Schließrichtung 18 greifen die Aufspannstifte 15 der ersten Formhälfte 11 in Bohrungen 16 ein, die in der zweiten Formhälfte 12 ausgebildet sind.

In Figur 2 ist die erste Formhälfte 11 mit einer auf den Aufnahmestiften 15 aufgespannten flexiblen ersten Lage 5 mit angeformten Kernteil 4 dargestellt, wobei das Kernteil 4 auf einer Außenseite 5A der ersten Lage 5 angeformt wurde. In der späteren Schuheinlage wird gemäß diesem Ausführungsbeispiel das Kernteil 4 Teil der Unterseite 9 der Schuheinlage 1 bilden und kann beispielsweise von einem Schuhorthopädie-Techniker individuell an den Träger der Schuheinlage angepasst werden. Für einen Fachmann ist es ersichtlich, dass entweder die Aufnahmestifte 15 oder die Aufspannlöcher 17 in der ersten Lage 5 so ausgebildet sind, dass die oftmals dreidimensionale Ausbildung des Kernteils 4 im Spritzgussprozess entweder über eine bevorzugt elastische Beweglichkeit der Aufnahmestifte 15 senkrecht zur Schließrichtung oder über eine elastische Verformbarkeit des Materials der ersten Lage 5 erfolgt, wobei beispielsweise die zweite formgebende Oberfläche 14 beim Schließen des Spritzgusswerkzeuges 10 die erste Lage 5 in eine dreidimensionale Form zwingt, bevor Spritz-Kunststoff in die Kavität der Spritzgussform 10 eingespritzt wird.

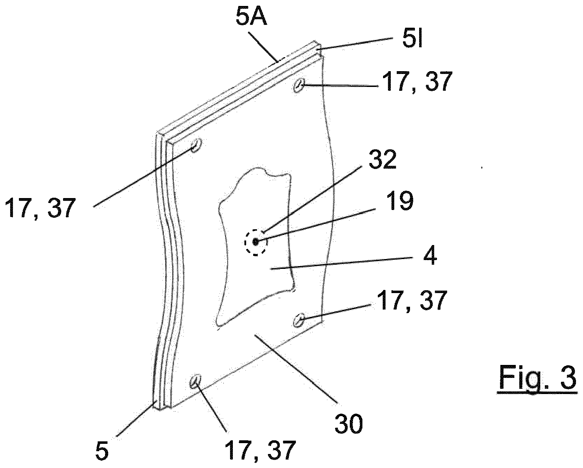

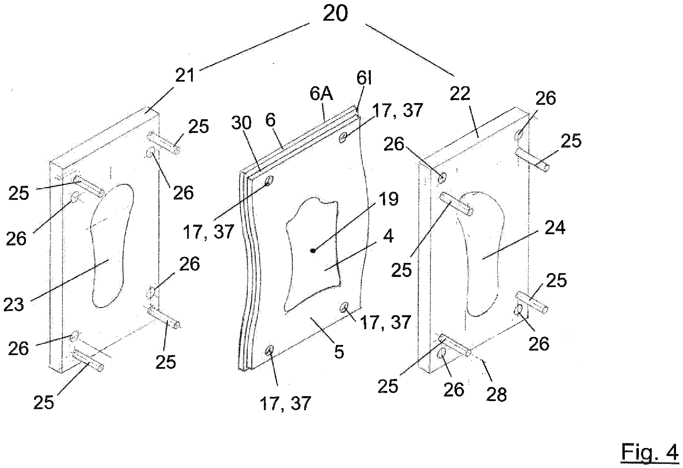

Figur 3 zeigt schematisch eine weitere Ausführungsform einer flexiblen ersten Lage 5 mit einem daran ausgebildeten Kernteil 4 in einer perspektivischen Darstellung, wobei hier vor dem Spritzformen des Kernteils 4 eine Verstärkungslage 30 zwischen die formgebende Oberfläche der Anspritzseite des Spritzgusswerkzeuges 10- hier die Formhälfte 11 - und der Seite der flexiblen ersten Lage 5 in das Spritzgusswerkzeug 10 eingelegt/eingehängt wird, sodass diese Verstärkungslage 30 über den Spritzgusskunststoff an die Innenseite 5l der ersten Lage 5 angeheftet wird. Je nach Beschaffenheit und Ausgestaltung des Materiales der Verstärkungslage 30, kann es erforderlich sein, die Verstärkungslage 30 im Bereich des Anspritzpunktes 19 für das Anformen des Kernteils 4 an die erste Lage 5 mit einer Freimachung 32 zu versehen, damit das Spritzgussmaterial ungehindert durch die Verstärkungslage 30 hindurchtreten kann. Bevorzugt wird die Verstärkungslage 30 mit Aufspannlöcher 37 in die erste Formhälfte 11 des Spritzgusswerkzeuges 10 ebenfalls an den Aufspannstiften 15 der ersten Formhälfte 11 eingehängt, wobei die Aufspannlöcher 37 der Verstärkungslage 30 bevorzugt mit den Aufspannlöcher 17 der ersten Lage 5 übereinstimmen. Somit können diese beiden Lagen 5, 30, die über das Kernteil 4 miteinander verbundenen sind, für den folgenden Verfahrensschritt des RIM-Schäumens zusammen über die Aufspannlöcher 17 und 37 auf Aufspannstifte 25 einer ersten Formhälfte 21 einer RIM-Schäumform 20 eingehängt und dort fixiert werden (vgl. Figur 4).

Ein Fachmann erkennt, dass auch ein separates Einhängen der ersten Lage 5 mit angeformten Kernteil 4, insbesondere für solche Ausführungsformen erfolgen kann, bei der das Kernteil 4 an der Außenseite 5A der flexiblen ersten Lage 5 angeformt ist, vgl. hierzu Ausführungen zu Figur 2. Hintergrund ist der, dass die Verstärkungslage 30 bevorzugt eine aus Fasern gebildete flexible Lage, beispielsweise ein Vlies ist, die vom Schaumkunststoff im RIM-Schäumvorgang durchdrungen wird und so den Schaumkunststoff in transversalen Richtungen verstärkt.

Figur 4 zeigt zwei Formhälften 21 und 22 einer RIM-Schäumform 20 im geöffneten Zustand, wobei die beiden Formhälften 21 und 22 zur Erleichterung der Darstellung von Einzelheiten analog zu Figur 1 nebeneinander angeordnet sind. Auch hier läuft die eigentliche Schließrichtung 28 der RIM-Schäumform in Längsrichtung der Aufspannstifte 25. Beide Formhälften 21, 22 der RIM-Schäumform 20 weisen sowohl Aufnahmestifte 25 als auch Aufnahmeöffnungen 26 für die Aufnahme der Aufnahmestifte 25 der jeweils anderen Formhälfte auf, wenn die RIM-Schäumform 20 geschlossen wird. Dabei erkennt der Fachmann, dass die Aufnahmestifte 25 der ersten Formhälfte 21 versetzt zu den Aufnahmeöffnungen 26 in der ersten Formhälfte 21 angeordnet sind, so dass bei einem Schließen des Formwerkzeugs 20 die Aufnahmestifte 25 einer Formhälfte 21 oder 22 in die Aufnahmeöffnungen 26 der jeweils anderen Formhälfte 22 oder 21 eingreifen können.

Ein Fachmann leitet insbesondere aus Figur 3 ab, wie dies nicht maßstabsgetreu beispielsweise in Figur 4 dargestellt ist, dass die erste flexible Lage 5 aus einem faserhaltiges Material aufgebaut sein kann, das sowohl für den Spritzguss-Kunststoff als auch den Schaumkunststoff - zumindest in seiner nicht-ausgehärteten Form - durchlässig ist. Somit kann die erste Lage 5 die Funktion einer Verstärkungslage 30 übernehmen; sie ist in diesem Fall jedoch als Unterdecke 3 nur noch bedingt geeignet, wenn die Unterseite 9 der Schuheinlage 1 frei von PU-Schaum sein soll. Hier bietet sich die Verwendung einer zweiten Lage 6 als Materiallage für eine Unterdecke 3 an, die dann zwischen eine formgebende Oberfläche 23 einer ersten Schäumformhälfte 21 einer RIM-Schäumform 20 und der ersten Lage 5 eingelegt wird und bevorzugt zumindest eine für den PU-Schaumkunststoff undurchlässige Schicht aufweist. Dabei ist es nicht erforderlich, dass die erste Lage 5 und die zweite Lage 6 über das spritzgegossene Kernteil 4 miteinander verbunden sind. Der in der geschlossenen Schäumform 20 erzeugte Schaumkunststoff quillt bei diesem Ausführungsbeispiel innerhalb der Kavität der Schäumform 20 durch vom Kernteil 4 nicht versteifte Bereiche der ersten Lage 5 und benetzt zumindest die vom Kernteil 4 nicht abgeschatteten Bereiche der Innenseite 6l der zweiten Lage 6. Dadurch wird eine Verbindung der ersten Lage 5 mit der zweiten Lage 6 über den Schaumkunststoff hergestellt. In Schießrichtung 28 der Schäumform 20 begrenzt die formgebende Oberfläche 24 die Kavität der Schäumform 20 bevorzugt derart, dass an der Oberseite 8 der Schuheinlage 1 ein orthopädisches Fußbett ausgebildet wird, wobei bevorzugt die der formgebenden Oberfläche 24 zugewandte Seite der ersten Lage 5 vollständig mit Schaumkunststoff bedeckt ist. Auf dieses orthopädische Fußbett kann dann, ggf. nach individueller Anpassung des Fußbettes, in einem weiteren Arbeitsschritt eine Oberdecke 2 aufgebracht werden.

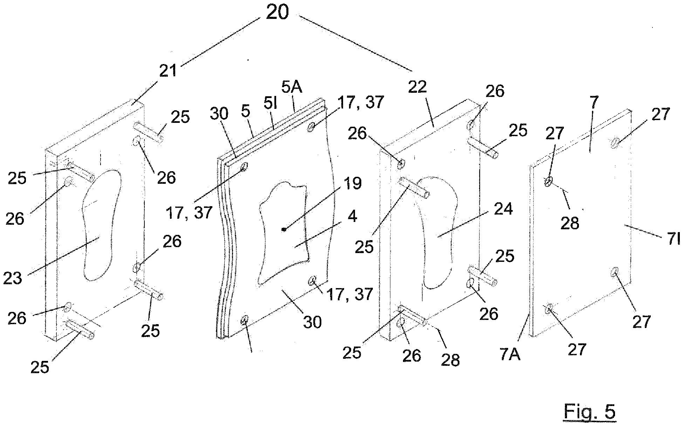

Wie oben bereits erläutert, ist dies eine mögliche Ausführungsform für die Anordnung einer Aufspannvorrichtung für die Aufnahme der ersten Lage 5 bzw. einer zweiten Lage 6 und ggf. einer dritten Lage 7. Figur 5 zeigt ebenfalls nicht maßstabsgetreu eine Ausführungsform mit einer die Oberdecke 2 in RIM-Schäumschritt anbindbaren dritten Lage 7. Im Unterschied zum Ausführungsbeispiel der Figur 4 wird die Unterdecke 2 durch die erste Lage 5 gemäß dem obig beschriebenen Ausführungsbeispiel der Figur 3 gebildet, an die zuvor im Spritzgussverfahren beim Anformen des Kernteils 4 an die erste Lage 5 eine Verstärkungslage 30 mit angeheftet wurde. Die einzelnen Lagen sind in der Figur 5 beabstandet von den jeweiligen Formhälften 21, 22 dargestellt. Dabei erkennt man, dass an der Innenseite der ersten Lage 5 ein Kernteil 4 angeformt ist, wobei die Außenseite 5A der ersten Lage 5, die spätere Unterseite 9 der Schuheinlage bildet, da sie der formgebenden Oberfläche 23 der ersten Formhälfte 21 der RIM-Schäumform 20 zugewandt ist. Das angespritzte Kernteil 4 ist auf der der formgebenden Oberfläche 23 abgewandten Innenseite 5l der ersten Lage 5 angeformt und mit einer Verstärkungslage 30 überspannt. Somit wird das Kernteil nach dem Schäumvorgang an der fertigen Schuheinlage 1 verdeckt sein. Des Weiteren wurde als Material für die erste Lage 5 ein zweischichtiges Material ausgewählt, dessen Innenseite 5l ein gewebeartiges Textil ist, das mit dem spritzgegossenen Kernteil 4 fest verbunden ist. Die Außenseite 5A der ersten Lage 5 wird durch eine für den nicht ausgehärteten Schaumkunststoff undurchlässige Sperrschicht 5U gebildet, die nicht notwendigerweise eine Verbindung mit dem Spritzgussmaterial eingegangen ist. Bevorzugt ist das Material der Sperrschicht 5U, welches die Unterseite 9 der späteren Schaumkunststoff-Schuheinlage 1 bildet, ein elastisches, insbesondere gummiartiges Material, das der Schuheinlage eine rutschfeste Unterseite 9 verleiht, damit die Schaumkunststoff-Schuheinlage 1 in einem Schuh, beispielsweise rutschfest, eingelegt werden kann.

Eine dritte Lage 7 ist über die zweite Formhälfte 22 der RIM-Schäumform 20 (hier die rechte Formhälfte 22) über die formgebende Oberfläche 24 aufgespannt. Dies erfolgt analog zu der Aufspannung der ersten Lage 5 vor der formgebenden Oberfläche 23 der ersten Formhälfte 21. Analog zu der für den Schaumkunststoff undurchlässigen Sperrschicht 5U auf der Außenseite 5A der ersten Lage 5 kann auch auf oder in der dritten Lage 7 eine derartige Schaumkunststoffundurchlässige Sperrschicht 7U angeordnet sein. Beispielsweise dann, wenn ein mehrschichtiges Material für die dritte Lage 7 verwendet wird und die Innenseite 7l aus einem Material gebildet wird, das für den Schaumkunststoff durchlässig ist. Soll zudem vermieden werden, dass Schaumkunststoff an die Oberseite 8 der Schuheinlage 1 gelangt, kann beispielsweise zwischen der Schicht für die Innenseite 7l und die Schicht für die Außenseite 7A, d.h. die Oberseite 8 der Schuheinlage 1, z.B. die Dekorseite, eine Schaumkunststoff-Sperrschicht 7U angeordnet werden.

Nach Aufspannen und Fixieren der ersten Lage 5 mit angehefteter Verstärkungslage 30 und der dritten Lage 7 auf die entsprechenden Schäumformhälften 21, 22 werden die zum Erzeugen des Schaumkunststoff erforderlichen Reaktionspartner beispielsweise vermischt mittels eines Mischkopfes in die RIM-Schäumform 20 eingebracht. In der geschlossenen Schäumform kann der sich bildendende Schaumkunststoff mit den Innenseiten 5l und 7l der ersten Lage 5 und der dritten Lage 7 verbinden und gleichzeitig durch Expansion des Schaumkunststoffs in der durch die Schäumformhälfte gebildeten Kavität einen Schaumkunststoff-Schuheinlage-Rohling 100 mit orthopädischem Fußbett ausbilden. Wie oben bereits angedeutet bildet der Schaumkunststoff ganz oder teilweise den Polsterkörper 31 der Schaumkunststoff-Schuheinlage. Schließt man gedanklich die in Figur 5 dargestellte Schäumform 20 und lässt den Schäumschritt ablaufen, so kann man nach dem Aushärten des PU-Schaumkunststoffs einen Schuheinlage-Rohling 100 aus der RIM-Schäumform 20 entnehmen, der sowohl auf der Unterseite 9 als auch auf der Oberseite 8 mit einer Unterdecke 3 bzw. einer Oberdecke 2 bezogen ist, wobei beide Decken mittels dem angeschäumten PU-Schaumkunststoff miteinander verbunden sind.

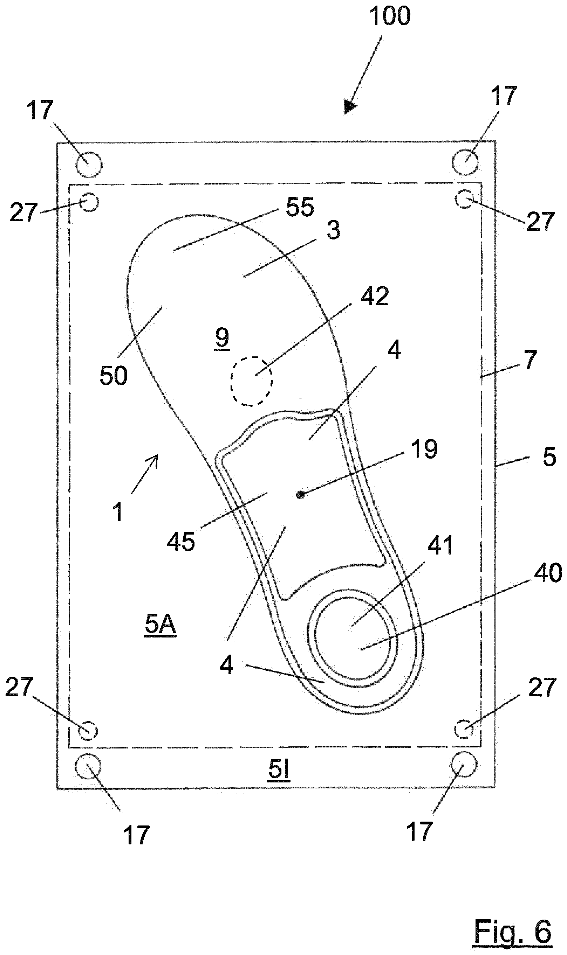

Ein aus der RIM-Schäumform 20 entnommener Schuheinlagerohling 100 ist in Figur 6, in einer schematischen Darstellung gezeigt, die der Übersichtlichkeit halber nur einen linken Schuheinlage-Rohling 100 zeigt. Wie oben im allgemeinen Teil der Erfindungsbeschreibung bereits ausgeführt, bietet sich bei der Ausführung des erfindungsgemäßen Verfahrens jedoch an, mit den jeweiligen Formwerkzeugen zumindest paarweise Schuheinlagenrohlinge 100 bzw. Schuheinlagen 1 herzustellen. Weiter zeigt der in Figur 6 dargestellte Schaumkunststoff-Schuheinlage-Rohling 100 ein auf der Außenseite 5A der ersten Lage 5, also auch auf der Unterseite 9 der Schuheinlage 1, ausgebildetes Kernteil 4, welches sich vom Mittelfußbereich 45 bis zum Fersenbereich 40 erstreckt. Dabei ist im Fersenbereich 40 des Kernteils 4 eine Ausnehmung 41 im Kernteil 4 vorgesehen, welche mit PU-Schaumkunststoff gefüllt ist, um beispielsweise den Fersensporn des Trägers der Schuheinlage 1 besser dämpfen zu können. Der die Ausnehmung 41 umgebende Bereich wird weiterhin von dem Sohlen-stabilisierenden Kernteil 4 unterstützt. Für einen Fachmann ist leicht ersichtlich, dass das Kernteil 4 auch auf weitere Bereiche, wie den Vorderfußbereich 50 oder den Zehenbereich 55, ausgedehnt werden kann, wobei dort ebenfalls Ausnehmungen im Kernteil 4 und/oder in der ersten Lage 5 vorhanden sein können, die mit Schaumkunststoff gefüllt sind.

Ein Fachmann erkennt weiter, dass mit dem erfindungsgemäßen Verfahren zusätzlich auch weitere Kernteile oder orthopädische Hilfsmittel, wie Pelotten oder ähnliches beispielsweise im Bereich der Zehengrundgelenke an die erste Lage 5 im Spritzgussverfahren angeformt oder an die zweite Lage 6 oder die dritte Lage 7 vor dem RIM-Schäumverfahren angebracht werden können, ohne vom Erfindungsgedanken abzuweichen. Bevorzugt werden Pelotten jedoch direkt im RIM-Schäumschritt durch den dort erzeugten Schaumkunststoff ausgebildet und müssen dann nicht als Einzelteile an der jeweiligen Lage 5, 6 oder 7 befestigt werden. Andererseits ermöglicht das Einlegen von Pelotten oder ähnlichen orthopädischen Hilfsmitteln eine stärkere Individualisierung der erfindungsgemäß hergestellten Schuheinlagen.

Weiter ist aus Figur 6 gut zu erkennen, dass ein oder mehrere Paare von Schuheinlagen 1, d.h. mehr als eine linke und eine rechte Schuheinlage gleichzeitig ausgebildet werden können. Hierzu müssen die in den Figuren 1,2, 4 und 5 dargestellten Formnester der Formwerkzeuge 10 und 20 entsprechend gespiegelt und vervielfältigt werden.

Zum Ausbilden der fertigen Schaumkunststoff-Schuheinlage 1 muss der in Figur 6 dargestellte Schuheinlage-Rohling 100 nur noch fachüblich in seiner Form/Kontur zugeschnitten werden, was bevorzugt durch Stanzen erfolgt, aber auch manuell geschehen kann. Im Sinne der Erfindung bedeutet fertige Schaumkunststoff-Schuheinlage 1, dass diese insoweit gebrauchsfertig ist, damit sie entweder direkt in einen Schuh eingelegt werden kann oder aber auch einem Schuhorthopäden zur Verfügung gestellt werden kann, damit dieser die Schaumkunststoff-Schuheinlage 1 individuell an den Träger der Schuheinlage anpassen kann.

Insgesamt wird mit dem erfindungsgemäßen Verfahren und der erfindungsgemäßen Anpassung der hierfür verwendeten Spritzgusswerkzeuge 10 sowie der verwendeten RIM-Schäumformen 20 ein einfaches sowie robustes Herstellverfahren bereitgestellt, mit dem hochqualitative, ggf. individuell anpassbare Schuheinlagen herstellbar sind. Dabei werden insbesondere die kostspieligen und hohe Sorgfalt erfordernden Verfahrensschritte zumindest für das Beziehen eines PU-Schaumkunststoff-Rohlings mit einer Unterdecke und/oder einer Oberdecke vermieden. Gleichzeitig kann erfindungsgemäß eine dünne Schuheinlage 1 hergestellt werden, die die biometrischen Verhältnisse eines Schuhs, in den die erfindungsgemäße Schuheinlage 1 eingelegt werden soll, nur minimal beeinträchtigt. Dabei sind sämtliche fachübliche Abwandlungen, insbesondere zur Aufnahme und Anordnung der einzelnen Lagen in den einzelnen Formhälften der Formwerkzeuge, als auch der Anordnung der Formhälften bzw. formgebenden Oberflächen in den Formhälften vom Erfindungsgedanken umfasst oder können mit den hier aufgezeigten Ausführungsformen ohne Weiteres in vielfältiger Weise kombiniert werden.

**Bezugszeichenliste**

| | | | |
|---|---|---|---|
| 1 | Schuheinlage | 20 | RIM-Schäumform |
| 2 | Oberdecke | 21 | Erste Schäumformhälfte |
| 3 | Unterdecke | 22 | Zweite Schäumformhälfte |
| 4 | Kernteil | 23 | Formgebende Oberfläche |
| 5 | Erste Lage | 24 | Formgebende Oberfläche |
| 5A | Außenseite erste Lage | 25 | Aufspannstift |
| 5I | Innenseite erste Lage | 26 | Bohrung |
| 5U | Sperrschicht erste Lage | 27 | Aufspannlöcher zweite Lage |
| 6 | Zweite Lage | 28 | Schließrichtung |
| 6A | Außenseite zweite Lage | | |
| 6I | Innenseite zweite Lage | 30 | Verstärkungslage |
| 6U | Sperrschicht zweite Lage | 31 | Polsterkörper |
| 7 | Dritte Lage | 32 | Freimachung |
| 7A | Außenseite dritte Lage | | |
| 7I | Innenseite dritte Lage | 37 | Aufspannlöcher dritte Lage |
| 7U | Sperrschicht dritte Lage | | |
| 8 | Oberseite Schuheinlage | | |
| 9 | Unterseite Schuheinlage | 40 | Fersenbereich |
| | | 41 | Ausnehmung Fersenbereich |
| 10 | Spritzgusswerkzeug | 42 | Pelotte |
| 11 | Erste Formhälfte | | |
| 12 | Zweite Formhälfte | 45 | Mittelfußbereich |
| 13 | Formgebende Oberfläche | 46 | Ausnehmung Mittelfußbereich |
| 14 | Formgebende Oberfläche | | |
| 15 | Aufspannstift | 50 | Vorderfußbereich |
| 16 | Bohrung | 51 | Ausnehmung Vorderfußbereich |
| 17 | Aufspannlöcher erste Lage | | |
| 18 | Schließrichtung | 55 | Zehenbereich |
| 19 | Anspritzpunkt | | |
| | | 100 | Schuheinlagen-Rohling |

## Patentansprüche

1. Verfahren zum Herstellen einer orthopädischen Schaumkunststoff-Schuheinlage (1) mit einem im RIM-Schäumverfahren an der Oberseite (8) erzeugten orthopädischen Fußbett, einer Unterseite (7) sowie mit einem die Schaumkunststoff-Schuheinlage (1) stabilisierenden, spritzgegossenen Kernteil (4), aufweisend die folgenden Schritte:
- Fixieren einer ersten Lage (5) eines flexiblen Materials, dessen Flächenabmessungen größer sind als die der fertigen Schaumkunststoff-Schuheinlage (1), in einem Spritzgusswerkzeug (10);
- Anformen mittels Spritzgießen des Kernteils (4) in einem Teilbereich der ersten Lage (5) derart, dass das Kernteil (4) fest mit der ersten Lage (5) verbunden bleibt und den Teilbereich der ersten Lage (5) versteift;
- Übernahme der Position des Kernteils mittels der ersten Lage (5), an der es angespritzt ist, von der Spritzgussform zu einer RIM-Schäumform (20);
- Fixieren der ersten Lage (5) mit dem daran angeformten Kernteil (4) auf einer ersten Schäumformhälfte (21) der RIM-Schäumform (20) derart, dass das Kernteil (4) mittels der ersten Lage (5) in einer orthopädisch vorbestimmten Position gehalten wird;
- Einbringen der den PU-Schaumkunststoff erzeugenden Komponenten in die RIM-Schäumform (20) derart, dass die Seite (5l) der ersten Lage (5), die einer formgebenden Oberfläche (23) der ersten Schäumformhälfte (21) abgewandt ist, durch den im RIM-Schäumverfahren erzeugten Schaumkunststoff angeschäumt und gleichzeitig das Fußbett auf der Oberseite (8) durch den Schaumkunststoff ausgebildet wird;
- Aushärten lassen des Schaumkunststoffs in der geschlossenen RIM-Schäumform (20);
- Entnahme des Schuheinlage-Rohlings (9) aus der RIM-Schäumform (20) nach dem Aushärten; und
- Zuschnitt des Schuheinlage-Rohlings (9) zum Ausbilden der Schaumkunststoff - Schuheinlage (1).

2. Verfahren nach Anspruch 1, wobei eine Seite des Materials der ersten Lage (5) vor dem Spritzgießen mit einer für den flüssigen Spritzguss-Kunststoff undurchlässigen Schicht versehen wird.

3. Verfahren nach Anspruch 1 oder 2, wobei eine Seite der ersten Lage (5) vor dem Einbringen der den Schaumkunststoff erzeugenden Komponenten in die RIM-Schäumform (20) mit einer für den Schaumkunststoff undurchlässigen Sperrschicht (5U) versehen wird.

4. Verfahren nach Anspruch 1 oder 2, wobei eine zweite Lage (6) eines flexiblen Materials, welche eine für den Schaumkunststoff undurchlässige Sperrschicht (6U) aufweist, vor dem Einlegen und Fixieren der ersten Lage (5) in der ersten Schäumformhälfte (21) derart eingelegt und fixiert wird, dass eine der formgebenden Oberfläche (23) der ersten Schäumformhälfte (21) zugewandte Außenseite (6A) der zweiten Lage (6) nach dem Schäumen zumindest teilweise die Unterseite (9) der Schuheinlage (1) bildet.

5. Verfahren nach einem der Ansprüche 1 bis 4, wobei die Seite der ersten Lage (5) oder die der zweiten Lage (6), die der formgebenden Oberfläche (23) der ersten Schäumformhälfte (21) zugewandt ist, eine Schicht aufweist, die Ausnehmungen (41, 46, 51) in der ersten Lage (5) und /oder in der zweiten Lage (6) überdeckt.

6. Verfahren nach einem der Ansprüche 1 bis 5, wobei vor dem Einbringen der Komponenten in die RIM-Schäumform (20), die den Schaumkunststoff erzeugen, eine dritte Lage (7) eines die Oberdecke (2) bildenden flexiblen Materials in die zweite Schäumformhälfte (22) der RIM-Schäumform (20) derart eingelegt und fixiert wird, dass eine die Oberseite (8) der Schuheinlage (1) bildende Außenseite (7A) der dritten Lage (7) der formgebenden Oberfläche (24) der zweiten Schäumformhälfte (22) zugewandt ist.

7. Verfahren nach Anspruch 6, wobei die dritte Lage (7) vor dem Einbringen der den Schaumkunststoff erzeugenden Komponenten in die RIM-Schäumform (20) mit einer für den nicht-ausgehärteten Schaumkunststoff undurchlässigen Schicht (7U) versehen wird.

8. Verfahren nach einem der Ansprüche 1 bis 7, wobei die für den Schaumkunststoff undurchlässige Sperrschicht (5U, 6U, 7U) auf die Außenseite (5A, 6A, 7A) der ersten Lage (5) und/oder der zweiten Lage (6) und/oder der dritten Lage (7) aufgebracht wird.

9. Verfahren nach einem der Ansprüche 1 bis 8, wobei auf die erste Lage (5) auf die Seite, welche der Oberseite (8) der späteren Schuheinlage (1) zugewandt ist, vor dem Spritzgießen eine für den flüssigen Spritzguss-Kunststoff durchlässige Verstärkungslage (30) in die erste Formhälfte (11) eingelegt und dort fixiert wird.

10. Verfahren nach Anspruch 9, wobei die Verstärkungslage (30) im Bereich des Anspritzpunktes (19) für das Kernteil (4) eine Freimachung (32) aufweist.

11. Verfahren nach einem der Ansprüche 1 bis 8, wobei auf die erste Lage (5) oder die dritte Lage (7), vor dem RIM-Schäumen eine für den nicht-ausgehärteten Schaumkunststoff durchlässige Verstärkungslage (30) in die jeweilige Schäumformhälfte (21, 22) eingelegt und dort fixiert wird.

12. Verfahren nach einem der Ansprüche 1 bis 11, wobei die erste Lage (5), die optionale zweite Lage (6) und die optionale dritte Lage (7) sowie die optionale Verstärkungslage (30) in ihren Eck- und/oder Umfangsbereichen mit Aufspannlöcher (17, 27, 37) versehen sind, mittels denen die erste Lage (5), die optionale zweite Lage (6) und die optionale dritte Lage (7) sowie die optionale Verstärkungslage (30) auf Aufspannstifte (15, 25), die in den entsprechenden Eck- und/oder Umfangsbereichen der Formhälften (11, 12, 21, 22) des Spritzgusswerkzeuges (10) und der RIM-Schäumform (20) angeordnet sind, aufgespannt werden können, wobei die Aufspannstifte (15, 25) der jeweils einen Formhälfte (11, 12, 21, 22) in dafür vorgesehene Bohrungen (16, 26) der anderen, zugehörigen Formhälfte (11, 12, 21, 22) beim Schließen der Spritzgussform (10) bzw. der RIM-Schäumform (20) eingreifen.

13. Orthopädische Schaumkunststoff-Schuheinlage (1) mit einem aus Schaumkunststoff gebildeten Polsterkörper (31) und einem auf der Oberseite (8) der Schuheinlage (1) im RIM-Verfahren ausgebildeten, orthopädischen Fußbett sowie mit einem die Schaumkunststoff-Schuheinlage (1) stabilisierenden, im Spitzgussverfahren ausgebildeten Kernteil (4),
**dadurch gekennzeichnet, dass**
das Kernteil an eine zumindest teilweise die Unterseite (9) der Schaumkunststoff-Schuheinlage (1) bildende Unterdecke (3) angespritzt ist, wobei die Innenseite der Unterdecke (3) durch den im RIM-Schäumverfahren erzeugten Schaumkunststoff an den Polsterkörper (31) der Schaumkunststoff-Schuheinlage (1) angebunden ist.

14. Orthopädische Schaumkunststoff-Schuheinlage (1) gemäß Anspruch 13, wobei die Schaumkunststoff-Schuheinlage (1) ferner eine Oberdecke (2) aufweist, die mit ihrer Innenseite (71) ebenfalls mittels des Schaumkunststoffs an den Polsterkörper der Schaumkunststoff-Schuheinlage (1) angebunden ist.

15. Orthopädische Schaumkunststoff-Schuheinlage (1) gemäß Anspruch 13 oder 14, wobei die Unterdecke (3) und/oder die Oberdecke (2) das Kernteil (4) zumindest teilweise überdecken.

16. Orthopädische Schaumkunststoff-Schuheinlage (1) gemäß Anspruch 13, wobei das Kernteil (4) zumindest teilweise die Unterseite (9) der Schuheinlage (1) bildet.

17. Orthopädische Schaumkunststoff-Schuheinlage (1) gemäß einem der Ansprüche 13 bis 16, wobei die Unterdecke (3) die Unterseite (9) der Schuheinlage (1) nur teilweise bedeckt.

18. Orthopädische Schaumkunststoff-Schuheinlage (1) gemäß einem der Ansprüche 13 bis 17, wobei die Unterdecke (3) Ausnehmungen (41, 46, 51) im Fersenbereich (40), im Mittelfußbereich (45), im Vorderfußbereich (50) und/oder im Zehenbereich (55) aufweist, durch die der PU-Schaumkunststoff und/oder das Kernteil (4) sichtbar ist.

19. Orthopädische Schaumkunststoff-Schuheinlage (1) gemäß einem der Ansprüche 13 bis 18, wobei die Oberdecke (2) ein Leder, ein Kunstleder, Alcantára, ein Textil, ein Vlies, ein Kunststoff, ein Kautschuk oder ein ähnliches, flexibles Material ist, das auch aus den zuvor genannten Materialien zusammengesetzt sein kann.

20. Orthopädische Schaumkunststoff-Schuheinlage (1) gemäß einem der Ansprüche 13 bis 19, wobei die Unterdecke (3) ein Leder, ein Kunstleder, Alcantára, ein Textil, ein Vlies, ein Kunststoff, ein Kautschuk oder ein ähnliches, flexibles Material ist, das auch aus den zuvor genannten Materialien zusammengesetzt sein kann.

21. Orthopädische Schaumkunststoff-Schuheinlage (1) gemäß einem der Ansprüche 13 bis 20, wobei zwischen Oberdecke (2) und Unterdecke (3) eine für den nicht-ausgehärteten Schaumkunststoff durchlässige Verstärkungslage (30) angeordnet ist, die aus einem Textil, einem Vlies, einem Faserwerkstoff oder einer Zusammensetzung davon gebildet ist.

22. Orthopädische Schaumkunststoff-Schuheinlage (1) gemäß einem der Ansprüche 13 bis 21, wobei die Oberdecke (2) und/oder die Unterdecke (3) zusätzlich eine für den nicht-ausgehärteten Schaumkunststoff undurchlässige Schicht aufweist.

23. Orthopädische Schaumkunststoff-Schuheinlage (1) gemäß Anspruch 22, wobei die für den nicht-ausgehärteten Schaumkunststoff undurchlässige Schicht die Außenseite der Oberdecke (2) und/oder der Unterdecke (3) bildet, d.h. zumindest teilweise die Oberseite (8) und/oder die Unterseite (9) der Schuheinlage (1) bildet.

24. Orthopädische Schaumkunststoff-Schuheinlage (1) gemäß einem der Ansprüche 13 bis 23, wobei das Kernteil (4) aus einem thermoplastischen Kunststoff spritzgegossen ist, bevorzugt aus Polypropylen (PP) oder Polyethylen (PE).

25. Orthopädische Schaumkunststoff-Schuheinlage (1) gemäß einem der Ansprüche 13 bis 23, wobei das Kernteil (4) aus einem thermoelastischen Kunststoff spritzgegossen ist.

## Claims

1. Method for manufacturing an orthopaedic foam plastic shoe inlay (1) with an orthopaedic footbed produced on the upper side (8) by means of the RIM foaming method, a lower side (9) and an injection-moulded core part (4) stabilizing the foam plastic shoe inlay (1), comprising the following steps:
- fixing of a first layer (5) of a flexible material, the surface dimensions of which are greater than those of the finished foam plastic shoe inlay (1), in an injection moulding tool (10);
- forming of the core part (4) in a partial section of the first layer (5) by means of injection moulding in such a way that the core part (4) remains firmly connected to the first layer (5) and stiffens the partial section of the first layer (5);
- transfer of the position of the core part by means of the first layer (5), at which it is injected, from the injection mould to an RIM foaming mould (20);
- fixing of the first layer (5) to the core part (4) moulded thereon on a first foam mould half (21) of the RIM foaming mould (20) in such a way that the core part (4) is held in an orthopaedically predetermined position by means of the first layer (5);
- insertion of the components producing the PU foam plastic into the RIM foaming mould (20) in such a way that the side (51) of the first layer (5) facing away from a shaping surface (23) of the first foaming mould half (21) is foamed by the foam plastic produced by means of the RIM foaming method, with the footbed being formed on the upper side (8) by the foam plastic at the same time;
- allowing the foam plastic to harden in the closed RIM foaming mould (20);
- removal of the shoe inlay blank (100) from the RIM foaming mould (20) after hardening; and
- cutting of the shoe inlay blank (100) to form the foam plastic shoe inlay (1).

2. Method according to Claim 1, whereby one side of the material of the first layer (5) is provided with a layer impermeable to the liquid injection-mould plastic prior to injection moulding.

3. Method according to Claim 1 or 2, whereby one side of the first layer (5) is provided with a barrier layer (5U) impermeable to the foamed plastic before the insertion of the components producing the foamed plastic into the RIM foaming mould (20).

4. Method according to Claim 1 or 2, whereby a second layer (6) of a flexible material which has a barrier layer (6U) impermeable to the foamed plastic is inserted and fixed in the first foaming mould half (21) before the insertion and fixing of the first layer (5) in the first foaming mould half (21) in such a way that an outer side (6A) of the second layer (6) facing the shaping surface (23) of the first foaming mould half (21) at least partially forms the lower side (9) of the shoe inlay (1) after foaming.

5. Method according to one of Claims 1 to 4, whereby the side of the first layer (5) or that of the second layer (6) facing the shaping surface (23) of the first foaming mould half (21) has a layer which covers recesses (41, 46, 51) in the first layer (5) and/or in the second layer (6).

6. Method according to one of Claims 1 to 5, whereby before the components producing the foamed plastic are inserted into the RIM foaming mould (20), a third layer (7) of a flexible material forming the top cover (2) is inserted and fixed in the second foaming mould half (22) of the RIM foaming mould (20) in such a way that an outer side (7A) forming the upper side (8) of the shoe inlay (1) is facing the third layer (7) of the shaping upper surface (24) of the second foaming mould half (22).

7. Method according to Claim 6, whereby the third layer (7) is provided with a layer (7U) impermeable to the non-hardened foamed plastic before insertion of the components producing the foamed plastic into the RIM foaming mould (20).

8. Method according to one of Claims 1 to 7, whereby the barrier layer (5U, 6U, 7U) impermeable to the foamed plastic is applied to the outer side (5A, 6A, 7A) of the first layer (5) and/or the second layer (6) and/or the third layer (7).

9. Method according to one of Claims 1 to 8, whereby a reinforcement layer (30) permeable to the liquid injection-mould plastic is placed and fixed on the first layer (5), on the side facing the upper side (8) of the later shoe insert (1) before injection moulding.

10. Method according to Claim 9, whereby the reinforcement layer (30) exhibits an opening (32) in the area of the injection point (19) for the core part (4).

11. Method according to one of Claims 1 to 8, whereby on the first layer (5) or the third layer (7), a reinforcement layer (30) permeable to the non-hardened foamed plastic is inserted and fixed in the respective foam mould half (21, 22).

12. Method according to one of Claims 1 to 11, whereby the first layer (5), the optional second layer (6) and the optional third layer (7) as well as the optional reinforcement layer (30) are provided in their corner and/or peripheral areas with clamping holes (17, 27, 37) by means of which the first layer (5), the optional second layer (6) and the optional third layer (7) as well as the optional reinforcement layer (30) can be mounted on clamping pins (15, 25) which are arranged in the relevant corner and/or peripheral areas of the mould halves (11, 12, 21, 22) of the injection moulding tool (10) and of the RIM foaming mould (20), whereby the clamping pins (15, 25) of one mould half (11, 12, 21, 22) can engage in each case in holes (16, 26) provided for this purpose in the other corresponding mould half (11, 12, 21, 22) when the injection mould tool (10) or RIM foaming mould (20) is closed.

13. Orthopaedic foam plastic shoe inlay (1) with a cushioning body (31) formed from foam plastic and an orthopaedic footbed formed on the upper side (8) of the shoe inlay (1) by means of the RIM method and with a core part (4) stabilizing the foam plastic shoe inlay (1) and formed by the injection moulding method,
**characterized in that**
the core part is injection-moulded onto a bottom cover (3) forming at least partially the lower side (9) of the foam plastic shoe inlay (1), whereby the inner side of the bottom cover (3) is bonded to the cushioning body (31) of the foam plastic shoe inlay (1) by the foam plastic produced by the RIM foaming process.

14. Orthopaedic foam plastic shoe inlay (1) according to Claim 13, whereby the foam plastic shoe inlay (1) further comprises a top cover (2) which is also connected with its inner side (71) to the cushioning body of the foam plastic shoe inlay (1) by means of the foam plastic.

15. Orthopaedic foam plastic shoe inlay (1) according to Claim 13 or 14, whereby the bottom cover (3) and/or the top cover (2) at least partially cover the core part (4)

16. Orthopaedic foam plastic shoe inlay (1) according to Claim 13, whereby the core part (4) at least partially forms the lower side (9) of the shoe inlay (1).

17. Orthopaedic foam plastic shoe inlay (1) according to one of Claims 13 to 16, whereby the bottom cover (3) only partially covers the lower side (9) of the shoe inlay (1).

18. Orthopaedic foam plastic shoe inlay (1) according to one of Claims 13 to 17, whereby the bottom cover (3) has recesses (41, 46, 51) in the heel area (40), in the metatarsal area (45), in the forefoot area (50) and/or in the toe area (55) through which the PU foam plastic and/or the core part (4) is visible.

19. Orthopaedic foam plastic shoe inlay (1) according to one of Claims 13 to 18, whereby the top cover (2) is a leather, artificial leather, Alcantara, textile, fleece, plastic, rubber or similar flexible material which can also be a composition of the aforementioned materials

20. Orthopaedic foam plastic shoe inlay (1) according to one of Claims 13 to 19, whereby the upper cover (3) is a leather, artificial leather, Alcantara, textile, fleece, plastic, rubber or similar flexible material which can also be a composition of the aforementioned materials

21. Orthopaedic foam plastic shoe inlay (1) according to one of Claims 13 to 20, whereby a reinforcement layer (30) permeable to the non-hardened foam plastic is arranged between the top cover (2) and the bottom cover (3), the reinforcement layer (30) being formed from a textile, a fleece, a fibre material or a composition thereof.

22. Orthopaedic foam plastic shoe inlay (1) according to one of Claims 13 to 21, whereby the top cover (2) and/or the bottom cover (3) additionally exhibits a layer impermeable to the non-hardened foam plastic.

23. Orthopaedic foam plastic shoe inlay (1) according to Claim 22, whereby the layer impermeable to the non-hardened foam plastic forms the outside of the top cover (2) and/or the bottom cover (3), i.e. at least partially forms the upper side (8) and/or the lower side (9) of the shoe inlay (1).

24. Orthopaedic foam plastic shoe inlay (1) according to one of Claims 13 to 23, whereby the core part (4) is injection-moulded from a thermoplastic, preferably polypropylene (PP) or polyethylene (PE).

25. Orthopaedic foam plastic shoe inlay (1) according to one of Claims 13 to 23, whereby the core part (4) is injection-moulded from a thermoplastic.

## Revendications

1. Procédé de fabrication d'une semelle intérieure orthopédique (1) en mousse de plastique, avec une assise de pied orthopédique créée sur la face supérieure (8) via le procédé de moussage RIM, une face inférieure (9) et une partie centrale (4) moulée par injection qui stabilise la semelle intérieure (1) en mousse de plastique, comprenant les étapes suivantes :
- fixation d'une première couche (5) d'un matériau flexible, dont les dimensions des surfaces sont supérieures à celles de la semelle intérieure lage (1) en mousse de plastique, dans une pièce de moulage par injection (10) ;
- modelage de la partie centrale (4) par moulage par injection dans une zone partielle de la première couche (5), de sorte que la partie centrale (4) demeure solidement fixée à la première couche (5) et rigidifie la zone partielle de la première couche (5) ;
- transfert de la position de la partie centrale au moyen de la première couche (5), sur laquelle elle est moulée par injection, du moule d'injection vers un moule de moussage RIM (20) ;
- fixation de la première couche (5) avec la partie centrale (4) moulée sur cette dernière sur une première moitié (21) du moule de moussage RIM (20) de sorte à maintenir la partie centrale (4) dans une position prédéterminée à des fins orthopédiques à l'aide de la première couche (5) ;
- introduction des composants produisant la mousse de plastique PU dans le moule de moussage RIM (20), de telle sorte que la face (5l) de la première couche (5), qui est tournée vers une surface de moulage (23) de la première moitié (21) du moule de moussage, est moulée par la mousse de plastique produite durant le procédé de moussage RIM et que, en même temps, l'assise de pied est formée sur la partie supérieure (8) par la mousse de plastique ;
- durcissement de la mousse de plastique dans le moule de moussage RIM (20) fermé ;
- retrait de l'ébauche (100) de la semelle intérieure du moule de moussage RIM (20) après durcissement ; et
- découpage de l'ébauche (100) de la semelle intérieure pour obtenir la semelle intérieure (1) en mousse de plastique.

2. Procédé selon la revendication 1, une face du matériau de la première couche (5) étant pourvue d'une couche imperméable au plastique liquide du moulage par injection avant le moulage par injection.

3. Procédé selon la revendication 1 ou 2, une face de la première couche (5) étant pourvue d'une couche barrière (5U) imperméable à la mousse de plastique avant introduction des composants générant la mousse de plastique dans le moule de moussage RIM (20).

4. Procédé selon la revendication 1 ou 2, une deuxième couche (6) d'un matériau flexible, qui possède une couche barrière (6U) imperméable à la mousse de plastique, étant insérée et fixée avant la mise en place et la fixation de la première couche (5) de telle sorte qu'une face externe (6A) de la deuxième couche (6), tournée vers la surface de moulage (23) de la première moitié (21) du moule de moussage, forme après le moussage, au moins en partie, la face inférieure (9) de la semelle intérieure (1).

5. Procédé selon l'une quelconque des revendications 1 à 4, la face de la première couche (5) ou celle de la deuxième couche (6) tournée vers la surface de moulage (23) de la première moitié (21) du moule de moussage possédant une couche recouvrant des évidements (41, 46, 51) dans la première couche (5) et/ou dans la deuxième couche (6).

6. Procédé selon l'une quelconque des revendications 1 à 5, dans lequel, avant l'introduction des composants produisant la mousse de plastique dans le moule de moussage RIM (20), une troisième couche (7) d'un matériau flexible formant le revêtement supérieur (2) est inséré et fixé dans la seconde moitié (22) du moule de moussage RIM (20) de telle sorte qu'une face externe (7A) formant la face supérieure (8) de la semelle intérieure (1) fait face à la troisième couche (7) de la surface de moulage (24) de la seconde moitié (22) du moule de moussage.

7. Procédé selon la revendication 6, la troisième couche (7) étant pourvue d'une couche (7U) imperméable à la mousse de plastique non durcie avant introduction dans le moule de moussage RIM (20) des composants produisant la mousse de plastique.

8. Procédé selon l'une quelconque des revendications 1 à 7, la couche barrière (5U, 6U, 7U) imperméable à la mousse de plastique étant appliquée sur la face externe (5A, 6A, 7A) de la première couche (5) et/ou de la deuxième couche (6) et/ou de la troisième couche (7).

9. Procédé selon l'une quelconque des revendications 1 à 8, dans lequel sur la première couche (5) de la face tournée vers la face supérieure (8) de la future semelle (1), une couche de renforcement (30) perméable au plastique de moulage liquide est insérée et fixée dans la première moitié (11) du moule avant le moulage par injection.

10. Procédé selon la revendication 9, la couche de renforcement (30) comportant une évacuation (32) pour la partie centrale (4) dans la région du point d'injection (19).

11. Procédé selon l'une quelconque des revendications 1 à 8, dans lequel une couche de renforcement (30) perméable à la mousse de plastique non durcie est insérée et fixée dans la moitié respective (21, 22) du moule de moussage au-dessus de la première couche (5) ou de la troisième couche (7) avant le moussage RIM.

12. Procédé selon l'une quelconque des revendications 1 à 11, dans lequel la première couche (5), la deuxième couche facultative (6) et la troisième couche facultative (7) ainsi que la couche de renforcement facultative (30) sont pourvues dans leurs coins et/ou leurs zones périphériques de trous de fixation (17, 27, 37), au moyen desquels la première couche (5), la deuxième couche facultative (6) et la troisième couche facultative (7) ainsi que la couche de renforcement facultative (30) peuvent être fixées sur des chevilles de serrage (15, 25) disposées dans les coins et/ou zones périphériques respectives des moitiés du moule (11, 12, 21, 22) de la pièce de moulage par injection (10) et du moule de moussage RIM (20), les chevilles de serrage (15, 25) de chacune des moitiés de moule (11, 12, 21, 22) pénétrant dans les trous (16, 26) de l'autre moitié de moule correspondante (11, 12, 21, 22) lors de la fermeture de la pièce de moulage par injection (10) ou du moule de moussage RIM (20).

13. Semelle intérieure orthopédique (1) en mousse de plastique dotée d'un élément rembourré (31) et d'une assise de pied orthopédique formée sur la face supérieure (8) de la semelle intérieure (1) au cours du procédé RIM ainsi que d'une partie centrale (4) stabilisant la semelle intérieure (1) en mousse de plastique et formée au moyen du processus de moulage par injection,
**caractérisée en ce que**
la partie centrale est moulée par injection sur un revêtement inférieur (3) formant au moins partiellement la face inférieure (9) de la semelle intérieure (1) en mousse de plastique, la face interne du revêtement inférieur (3) étant reliée à l'élément rembourré (31) de la semelle intérieure (1) en mousse de plastique par la mousse de plastique produite lors du procédé de moussage RIM.

14. Semelle intérieure orthopédique (1) en mousse de plastique selon la revendication 13, la semelle intérieure (1) en mousse de plastique comportant en outre un revêtement supérieur (2) également relié à l'élément rembourré de la semelle intérieure (1) en mousse de plastique par sa face interne (71).

15. Semelle intérieure orthopédique (1) en mousse de plastique selon la revendication 13 ou 14, le revêtement supérieur (3) et/ou le revêtement inférieur (2) recouvrant au moins partiellement la partie centrale (4).

16. Semelle intérieure orthopédique (1) en mousse de plastique selon la revendication 13, la partie centrale (4) formant au moins partiellement la face inférieure (9) de la semelle intérieure (1).

17. Semelle intérieure orthopédique (1) en mousse de plastique selon l'une quelconque des revendications 13 à 16, le revêtement inférieur (3) couvrant uniquement en partie la face inférieure (9) de la semelle intérieure (1).

18. Semelle intérieure orthopédique (1) en mousse de plastique selon l'une quelconque des revendications 13 à 17, le revêtement inférieur (3) comportant des évidements (41, 46, 51) dans la zone du talon (40), dans la zone centrale du pied (45), dans la zone de l'avant-pied (50) et/ou dans la zone des orteils (55), à travers lesquels la mousse de plastique PU et/ou la partie centrale (4) est visible.

19. Semelle intérieure orthopédique (1) en mousse de plastique selon l'une quelconque des revendications 13 à 18, dans laquelle le revêtement supérieur (2) est un cuir, un similicuir, de l'alcantara, un textile, un non-tissé, une matière plastique, un caoutchouc ou un matériau souple similaire pouvant être également composé des matériaux précités.

20. Semelle intérieure orthopédique (1) en mousse de plastique selon l'une quelconque des revendications 13 à 19, dans laquelle le revêtement inférieur (3) est un cuir, un similicuir, de l'alcantara, un textile, un non-tissé, une matière plastique, un caoutchouc ou un matériau souple similaire pouvant être également composé des matériaux précités.

21. Semelle intérieure orthopédique (1) en mousse de plastique selon l'une des revendications 13 à 20, dans laquelle une couche de renforcement (30) perméable à la mousse de plastique non durcie est disposée entre le revêtement supérieur (2) et le revêtement inférieur (3), ladite couche étant constituée d'un textile, d'un non-tissé, d'une matière fibreuse ou d'une composition de ces matières.

22. Semelle intérieure orthopédique (1) en mousse de plastique selon l'une des revendications 13 à 21, le revêtement supérieur (2) et/ou le revêtement inférieur (3) comportant par ailleurs une couche imperméable à la mousse de plastique non durcie.

23. Semelle intérieure orthopédique (1) en mousse de plastique selon la revendication 22, la couche imperméable à la mousse de plastique non durcie formant la face externe du revêtement supérieur (2) et/ou du revêtement inférieur (3), c.-à-d. formant au moins en partie la face supérieure (8) ou la face inférieure (9) de la semelle intérieure (1).

24. Semelle intérieure orthopédique (1) en mousse de plastique selon l'une quelconque des revendications 13 à 23, la partie centrale (4) étant moulée par injection à partir d'un thermoplastique, de préférence à partir de polypropylène (PP) ou polyéthylène (PE).

25. Semelle intérieure orthopédique (1) en mousse de plastique selon l'une quelconque des revendications 13 à 23, la partie centrale (4) étant moulée par injection à partir d'un plastique thermoélastique.
